# EUROPEAN PATENT APPLICATION

(11) **EP 4 671 381 A2**
(43) Date of publication of application: **31.12.2025**
(21) Application number: 25198125.4
(22) Date of filing: 06.04.2017
(51) Int. Cl.: C12Q 1/686

(54) **METHODS FOR SYNTHESIS AND DETECTION OF NUCLEIC ACIDS**

(30) Priority: 06.04.2016 US 201662319151 P
(62) Divisional of application: 17718305.0
(71) Applicant: Life Technologies Corporation, Carlsbad, CA 92008 (US)
(72) Inventor: LE, Ferrier, Carlsbad (US); RAMAMOORTHI, Kalpith, Carlsbad (US); WILDE, Joyce, Carlsbad (US); FANTIN, Nicole, Carlsbad (US); LANG, Jordan, Carlsbad (US); DUPONT, David, Carlsbad (US); STEVENS, Junko, Carlsbad (US)
(74) Representative: Thermo Fisher Scientific- Life Sciences Solutions Group

(57) **Abstract**

Compositions, methods, and kits for synthesizing, detecting, and/or quantifying nucleic acids are provided herein. Embodiments comprise a nucleic acid amplification composition comprising a thermostable DNA polymerase and agents which improve nucleic acid synthesis, amplification, detection, and/or quantification of nucleic acid targets in a crude extract or crude lysate sample.

## Description

### CROSS-REFERENCE TO RELATED APPLICATIONS

This application claims the benefit of priority under 35 U.S.C. §119(e) to U.S. Provisional Patent Application No. 62/319,151, filed April 6, 2016, which is herein incorporated by reference in its entirety.

### FIELD

The present teachings are in the field of molecular biology and genetic analysis. The instant invention relates to compositions, methods and kits useful for the synthesis of nucleic acids. More specifically, compositions, methods and kits are provided for the amplification, detection, and/or quantitation of nucleic acid molecules, especially those having a low copy number and/or in the presence of inhibitors, such as in a crude lysate.

### BACKGROUND

In its simplest form, polymerase chain reaction (PCR) is an *in vitro* method for the enzymatic synthesis of specific DNA sequences using two oligonucleotide primers that hybridize to opposite strands and flank the region of interest in the target DNA. A repetitive series of reaction steps involving template denaturation, primer annealing, and the extension of the annealed primers by DNA polymerase results in the exponential accumulation of a specific fragment whose termini are defined by the 5' ends of the primers. PCR is capable of producing a selective enrichment of a specific DNA sequence by a factor of 10⁹. See, for example, U.S. Pat. Nos. 4,683,202, 4, 683,195, 4,800,159, and 4,965,188, and Saiki et al., 1985, Science 230:1350.

Commercially available PCR master mixes improve the efficiency and reduce the errors associated with the assembly of large number of PCR reactions required for high-throughput analysis. These master mixes contain a combination of reagents that will be common to all PCR reactions. For example, the master mix may contain a buffer, a salt such as MgCl₂, deoxynucleoside triphosphates (dNTPs), and a thermostable DNA polymerase. Typically, master mixes are manufactured and distributed as concentrated solutions or lyophilized powders which are subsequently diluted or dissolved when final reactions are assembled.

For accurate nucleic acid analysis, PCR master mixes should provide reliable, robust, and reproducible PCR results. Further, PCR master mixes should allow for detection of low copy number target nucleic acids. For example, many medical, diagnostic, and forensic applications depend on amplification of a particular DNA sequence in a sample in which the sequence is present in very low amounts. Also, many such applications rely on amplification of a nucleic acid sequence from a crude or unpurified sample, such as a cell or tissue crude lysate.

Accordingly, there is a need for PCR compositions which perform reliably with all types of nucleic acid samples, including crude sample preparations and/or those with targets in low amounts.

### SUMMARY

Provided herein are compositions, kits, and methods for the synthesis, detection, and/or quantitation of nucleic acids by polymerase-dependent nucleic acid synthesis, including, for example, using the polymerase chain reaction (PCR). In some embodiments, provided herein are compositions comprising a DNA polymerase, a salt, an antifoam agent, and a combination of a dNTP and a dNTP derivative.

In certain embodiments, the present teachings provide a composition comprising a thermostable DNA polymerase. In some embodiments, the thermostable DNA polymerase is selected from a Taq DNA polymerase; a Tne DNA polymerase; a Tma DNA polymerase; a Pfu DNA polymerase; a KOD DNA polymerase; a Tfl DNA polymerase; a Tth DNA polymerase; a Stoffel fragment DNA polymerase; a VENT^{™} DNA polymerase; a DEEPVENT^{™} DNA polymerase; and mutants, variants or derivatives thereof. In some embodiments the thermostable DNA polymerase is a Taq-derived DNA polymerase.

In certain embodiments, the present teachings provide a composition comprising a potassium salt, a magnesium salt, and/or a sodium salt. In some embodiments, the salt concentration is about 5 mM to about 200 mM.

In some embodiments, the composition comprises a silicone-based antifoam agent. In some embodiments, the silicone-based antifoam agent is selected from XIAMETER^{®} AFE-1010, AFE-1430, AFE-1510, AFE-1520, AFE-2210, Antifoam A, Antifoam B, Antifoam C, Antifoam H-10, Antifoam SE-15, Antifoam SE-35, Antifoam SO-25, Antifoam Y-30, and Antifoam 289. In some embodiments, the composition comprises a non-silicone-based antifoam agent. In some embodiments, the non-silicone-based antifoam agent is selected from organic sulfonates, polyethers, organic phosphates, acetylenic glycols, fluorocarbons, polyalkene polyamines, and polyalkyleneimine compounds, including without limitation Antifoam 204 and Antifoam O-30. In some embodiments the concentration of the antifoam agent is about 0.001% to 0.1%.

In some embodiments, the dNTP is selected from dGTP, dCTP, dATP and dTTP. In some embodiments the dNTP is dGTP. In some embodiments, the dGTP is at a concentration of 0.05 mM to 1.0 mM

In some embodiments the dNTP derivative is selected from 7-deaza-2-deoxy-GTP, 7-deaza-dATP, alpha-thio-dATP, alpha-thio-dTTP, alpha-thio-dGTP, and alpha-thio-dCTP. In some embodiments, the dNTP derivative is 7-deaza-2-deoxy-GTP. In some embodiments, the dNTP derivative is 7-deaza-2-deoxy-dGTP. In some embodiments, the 7-deaza-2-deoxy-dGTP is at a concentration of 0.05 mM to 1.0 mM.

In some embodiments, the composition comprises a dGTP and a 7-deaza-2-deoxy-dGTP at a ratio of about 1:2 to about 2:1. In some embodiments, the composition comprises a dGTP and a 7-deaza-2-deoxy-dGTP at a ratio of about 1:2 to about 1:10. In some embodiments, the composition comprises a dGTP and a 7-deaza-2-deoxy-dGTP at a ratio of about 2:1 to about 10:1. In some embodiments, the composition comprises a dGTP and a 7-deaza-2-deoxy-dGTP at a ratio of about 1:1.

In certain embodiments, the provided composition further comprises a PCR inhibitor blocking agent. In some embodiments, the PCR inhibitor blocking agent is a protein. In some embodiments, the PCR inhibitor blocking protein is selected from an albumin, a gelatin, and a combination thereof. In some embodiments, the composition comprises a gelatin and a serum albumin. In certain embodiments, the gelatin is selected from a bovine gelatin, a fish gelatin and a combination thereof. In certain embodiments, the bovine gelatin is at a concentration of 0.01% to 1.0% and/or said fish gelatin is at a concentration of 0.01% to 1.0%. In some embodiments, the serum albumin is a bovine serum albumin. In certain embodiments, the bovine serum albumin is at a concentration of 0.05 mg/ml to 5 mg/ml.

In certain embodiments, the provided composition further comprises a detergent. In some embodiment, the detergent is a non-ionic detergent. In some embodiments, the non-ionic detergent is selected from the group consisting of TRITON X-100^{®}, Nonidet P-40, TWEEN 80, Brij 30, Brij 35, and Brij 58. In some embodiments, the non-ionic detergent is a detergent other than Tween-20. In some embodiments, the non-ionic detergent is present in the composition at a concentration of .005% to 0.1%. In certain embodiments, the provided composition further comprises a passive reference control dye. In some embodiments, the passive reference control dye is a fluorescent dye. In some embodiments the passive fluorescent passive reference dye is a ROX dye or a MUSTANG PURPLE dye. In some embodiments, the passive reference dye is at a concentration of about 25 nM to about 500 nM.

In a certain embodiment, the present teaching provide a composition comprising a thermostable DNA polymerase, potassium chloride, an antifoam agent comprising silicon, dGTP and 7-deaza-2-deoxy-dGTP where the 7-deaza-2-deoxy-dGTP is at a concentration of about 0.05 mM to 1.0 mM, a non-ionic detergent other than Tween-20, bovine gelatin at a concentration of 0.01% to 1.0%, fish gelatin at a concentration of 0.01% to 1.0%, and bovine serum albumin at a concentration of 0.05 mg/ml to 5.0 mg/ml.

In certain embodiments, the present teachings provide methods for performing nucleic acid synthesis comprising contacting a composition provided herein with a biological sample comprising a target polynucleotide with a target-specific primer, in any order or combination of components, to form a reaction mixture and performing a nucleic acid synthesis reaction in the reaction mixture to form an amplification product. In some embodiments, the nucleic acid synthesis reaction is a polymerase chain reaction (PCR). In some embodiments the nucleic acid synthesis reaction is an amplification reaction. In some embodiments, the nucleic acid synthesis reaction is a primer extension reaction.

In some embodiments, the methods provided further comprise determining a genotype of the target polynucleotide using the amplification product. In some embodiments, the methods provided further comprise determining the copy number of the target polynucleotide using the amplification product.

In some embodiments of the provided methods, the run time of the PCR performed using the compositions or reactions mixtures as described herein is decreased compared to an equivalent PCR performed with a standard PCR reaction mix. In some embodiments, the PCR performed using the compositions or reaction mixtures as described herein produces an amplification product in a shorter period of time compared to an equivalent PCR performed with a standard PCR reaction mix.

In some embodiments, the PCR is a simplex PCR. In some embodiments, the PCR is a multiplex PCR. The term "simplex" or "simplex PCR" as used herein refers to an assay that provides for amplification of a single product within a reaction vessel. The product is primed using a distinct primer pair. A simplex reaction may further include a labeled probe specific for the amplified product, wherein the probe is detectably labeled with detectable moiety, such as a fluorescent dye. The term "multiplex" or "multiplex PCR" as used herein refers to an assay that provides for simultaneous amplification of two or more products within the same reaction vessel. Each product is primed using a distinct primer pair. A multiplex reaction may further include labeled probes specific to each product, wherein the probes are detectably labeled with different detectable moieties. In some embodiments, multiplex PCR includes those in which: (i) a multiplicity of targets are amplified in a single sample; two or more targets in one sample or (ii) multiple samples are simultaneously amplified; two targets in two different samples within a single reaction at substantially the same time.

In some embodiments of the provided methods, the biological sample is a lysate, such as a blood lysate or a lysate comprising oral-derived cells. In some embodiments, the biological sample is from saliva, urine, or serum. In some embodiments, the lysate is a crude lysate. In some embodiments, the biological samples comprises one or more PCR inhibitors including, including but not limited to IgG, hematin, heparin, EDTA, sodium citrate, or any combination thereof.

In certain embodiments, the present teachings provide kits comprising the compositions provided herein. In some embodiment, the kits further comprise a primer pair and/or a labeled probe specific for a nucleic acid synthesis (e.g., PCR amplification) product and/or detection of a DNA target.

In certain embodiments, the present teachings provide kits comprising the compositions provided herein, a control nucleic acid sample, and a primer pair and/or a labeled probe specific for a nucleic acid synthesis (e.g., PCR amplification) product and/or detection of a DNA target in the control nucleic acid sample.

The above described and other features are exemplified by the following figures and detailed description.

### BRIEF DESCRIPTION OF THE DRAWINGS

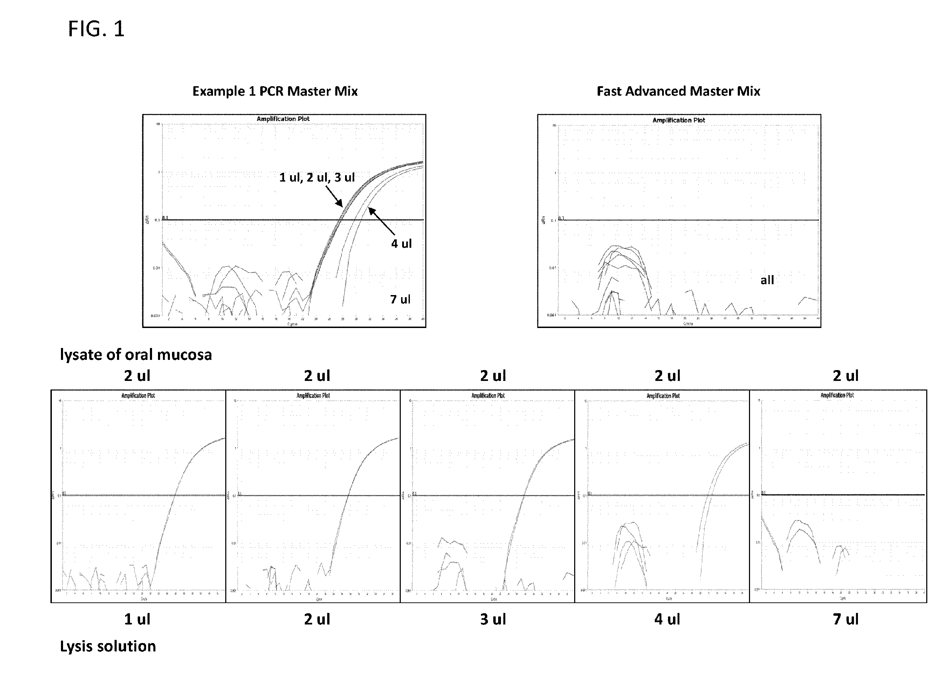

FIG. 1 shows graphs of Δ Rn as a function of PCR cycle for a two microliter sample of an oral mucosa crude lysate amplified in duplicate runs of the RNase P TaqMan^{™} Copy Number Assay using an embodiment of the compositions disclosed herein (left upper panel) and a commercially available PCR Master Mix (right upper panel). The lower panel shows individual graphs for the results with both master mixes at each volume of added lysis solution.

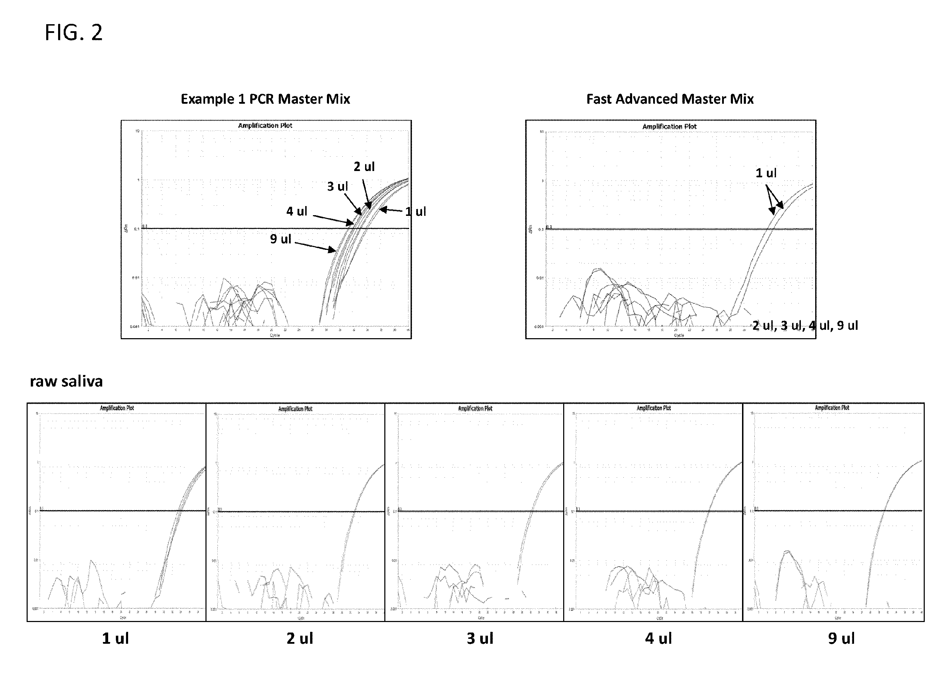

FIG. 2 shows graphs of Δ Rn as a function of PCR cycle for varying sample amounts of a raw saliva amplified in duplicate runs of the RNase P TaqMan^{™} Copy Number Assay using an embodiment of the compositions disclosed herein (left upper panel) and a commercially available PCR Master Mix (right upper panel). The lower panel shows individual graphs for the results with both master mixes at each volume of the raw saliva.

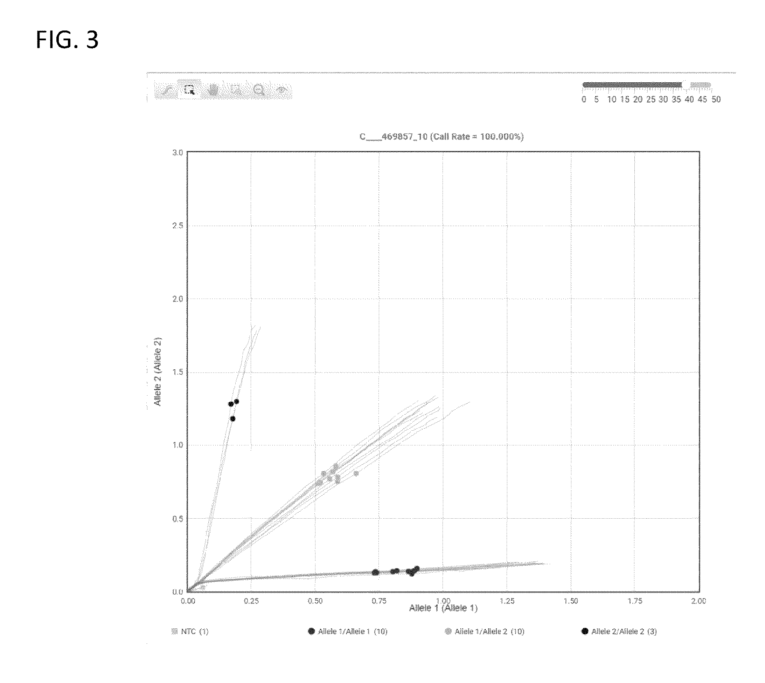

FIG. 3 shows a genotyping plot from assays perform on blood crude lysates using an embodiment of the compositions disclosed herein.

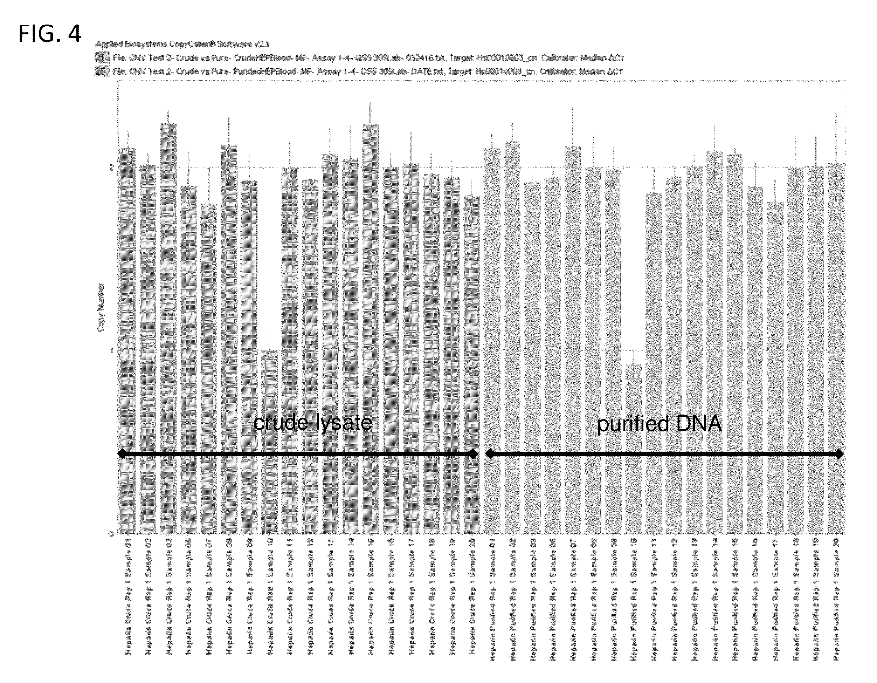

FIG. 4 is a graph of results from a copy number variation assay performed with blood crude lysate (left bars) and with corresponding purified DNA samples (right bars). The assays were performed with an embodiment of the compositions disclosed herein.

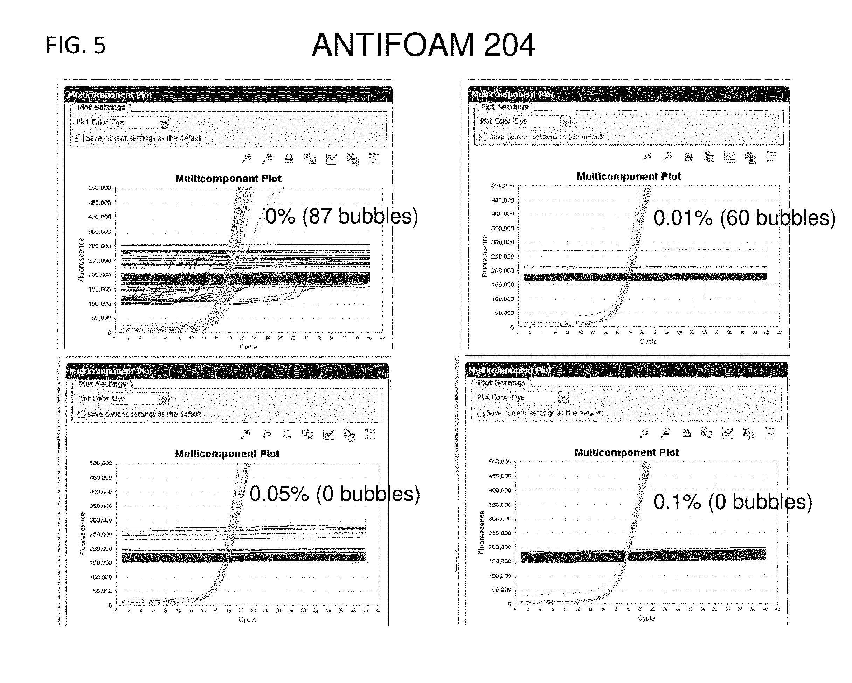

FIG. 5 shows graphs of SYBR^{®} Green fluorescence signal as a function of PCR cycle for reaction mixtures comprising varying amounts of anti-foam agent, Antifoam 204, at concentrations ranging from 0% to 0.1%. Reactions (10 uL) were run in 96 replicate on a 384-well microtiter plate using a ViiA 7 Real-Time PCR System.

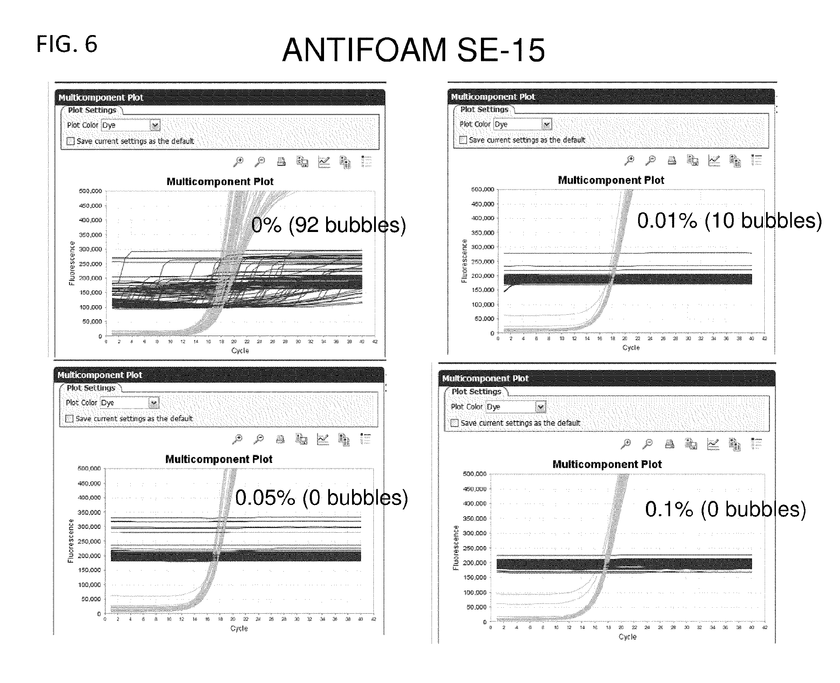

FIG. 6 shows graphs of SYBR^{®} Green fluorescence signal as a function of PCR cycle for reaction mixtures comprising varying amounts of anti-foam agent, SE-15, at concentrations ranging from 0% to 0.1%. Reactions (10 uL) were run in 96 replicate on a 384-well microtiter plate using a ViiA 7 Real-Time PCR System.

### DETAILED DESCRIPTION

In some embodiments, provided herein are compositions, methods, and kits for synthesis, detection, and/or quantitation of nucleic acids. In some embodiments, provided herein are compositions, methods, and kits for synthesis, detection, and/or quantitation of nucleic acids by polymerase-mediated nucleic acid synthesis, including but not limited to primer extension or polymerase chain reaction (PCR). In some embodiments, the compositions provided herein are particularly effective and demonstrate superior performance with crude or unpurified nucleic acid samples, such as crude lysates or crude extracts.

In some embodiments, the compositions disclosed herein are useful in a wide range of assays which involve detecting, quantitating, and/or characterizing target nucleic acids. For example, the compositions can be used in assays for genotyping, including without limitation single nucleotide polymorphism (SNP), microsatellite analysis, and other mutation genotyping, for copy number determination and variation analysis, for detecting gene expression, and for detecting small RNA expression (e.g., microRNA expression).

Kits comprising the compositions are also disclosed, as are methods of using the compositions and kits.

In one aspect, provided herein are assembled PCR master mix compositions which show superior performance sensitivity, accuracy, and specificity compared with standard PCR reagent mixes when run on standard PCR instrumentation. In some embodiments, use of the assembled PCR master mixes provided herein result in superior amplification, detection, and/or quantitation of nucleic acid target molecules present in crude or unpurified sample preparations as compared with use of standard PCR compositions. In some embodiments, use of the assembled PCR master mixes provided herein result in superior amplification, detection, and/or quantitation of nucleic acid target molecules present in low copy number in the sample preparation as compared with use of standard PCR master mix compositions.

In some embodiments, use of the assembled PCR compositions provided herein for genotyping results in improved sensitivity and specificity as compared with use of standard PCR compositions. In some embodiments, use of the assembled PCR compositions provided herein for gene expression analysis results in improved sensitivity values as compared with use of standard PCR compositions. In some embodiments, such assembled PCR compositions for genotyping or gene expression analysis using crude lysates or unpurified nucleic acid samples generates results comparable to the results obtained with corresponding purified nucleic acid samples.

In some embodiments, use of the assembled PCR compositions provided herein for copy number determination and/or copy number variation analysis results in improved sensitivity, accuracy, and specificity as compared with use of standard PCR compositions. In some embodiments, use of the assembled PCR compositions provided herein for copy number determination and/or copy number variation analysis results in higher copy number call rate and concordance as compared with use of standard PCR compositions. In some embodiments, use of the assembled PCR compositions provided herein for copy number determination and/or analysis improves lower copy number ranges (maximum to minimum) and null values as compared with use of standard PCR compositions. Such improvements in copy number variation analysis are found when using crude nucleic acid samples, such as crude lysates, or purified nucleic acid samples. In some embodiments, use of the assembled PCR compositions provided herein for analysis of copy number variation using crude lysates as nucleic acid samples generates results comparable to the results obtained with corresponding purified nucleic acid samples.

In some embodiments, the superior sensitivity of PCRs assembled with the compositions disclosed herein permits reduced PCR run times on either FAST or standard PCR instrumentation. In some embodiments, the PCR compositions are for use in simplex PCR and detection reactions. In some embodiments, the PCR compositions are for use in multiplex PCR and detection reactions. Typically, multiplex PCR reduces the running cost and sample usage by detecting multiplex targets simultaneously from a single reaction.

In some embodiments, provided herein are assembled compositions comprising a thermostable DNA polymerase, a salt, an antifoam agent, and dNTPs. In some embodiments, provided herein are assembled compositions comprising a thermostable DNA polymerase, a salt, an antifoam agent, and a combination of at least one dNTP and at least one dNTP derivative.

In some aspects, the compositions of the present teachings comprise one or more polymerases. Such polymerases can be any enzyme capable of replicating a DNA molecule. In some embodiments, the compositions may comprise a DNA-dependent DNA polymerase, an enzyme for reverse transcription (RNA-dependent DNA polymerase), and/or a combination of both types of enzymes. In some embodiments, a combination of DNA dependent DNA polymerases and/or a combination of RNA-dependent DNA polymerase can be present in the compositions disclosed herein.

In some embodiments, the polymerases as used herein are thermostable DNA polymerases. In some embodiments, the thermostable DNA polymerases as used herein are not irreversibly inactivated when subjected to elevated temperatures for the time necessary to effect destabilization of single-stranded nucleic acids or denaturation of double-stranded nucleic acids during nucleic acid synthesis or PCR amplification. Irreversible denaturation of the enzyme refers to substantial loss of enzyme activity. Preferably a thermostable DNA polymerase will not irreversibly denature at about 90°-100°C under conditions such as is typically required for PCR amplification.

DNA polymerases in accordance with the present teachings can be isolated from natural or recombinant sources, by techniques that are well-known in the art (see, e.g., PCT Publication Nos. WO 92/06200; WO 96/10640; U.S. Patent Nos. 5,455,170; 5,912,155; and 5,466,591, the disclosures of which are fully incorporated herein by reference in their entireties), from a variety of thermophilic bacteria that are available commercially (for example, from American Type Culture Collection, Rockville, Md.) or can be obtained by recombinant DNA techniques (see, e.g., PCT Publication No. WO 96/10640 and U.S. Patent No. 5,912,155). Suitable for use as sources of thermostable polymerases or the genes thereof for expression in recombinant systems are, for example, the thermophilic bacteria Thermus thermophilus, Thermococcus litoralis, Pyrococcus furiosus, Pyrococcus woosii and other species of the Pyrococcus genus, Bacillus sterothermophilus, Sulfolobus acidocaldarius, Thermoplasma acidophilum, Thermus flavus, Thermus ruber, Thermus brockianus, Thermotoga neapolitana, Thermotoga maritima and other species of the Thermotoga genus, and Methanobacterium thermoautotrophicum, and mutants, variants or derivatives thereof. In some embodiments, the compositions, methods, and kits provided herein comprise thermostable DNA polymerases selected from the group consisting of Taq DNA polymerase, Tne DNA polymerase, Tma DNA polymerase, Tfi DNA polymerase, Pfu DNA polymerase, Pwo DNA polymerase, VENT^{™} DNA polymerase, DEEPVENT^{™} DNA polymerase, mutants or derivatives thereof having DNA polymerase activity, and any combination of the foregoing. Taq DNA polymerase and mutant forms thereof are commercially available, for example, from Life Technologies (Carlsbad, CA), or can be isolated from their natural source, the (e.g., from the thermophilic bacterium Thermus aquaticus for Taq polymerase), as described previously (see, e.g., U.S. Patent Nos. 4,889,818 and 4,965,188, the disclosures of which are incorporated herein by reference in their entireties). Tne DNA polymerase can be isolated from its natural source, the thermophilic bacterium Thermotoga neapolitana (see, e.g., PCT Publication No. WO 96/10640 and U.S. Patent No. 5,912,155), and Tma DNA polymerase can be isolated from its natural source, the thermophilic bacterium Thermotoga maritima (see, e.g., U. S. Patent No. 5,374,553, the disclosure of which is incorporated herein by reference in its entirety). Exemplary thermostable polymerases include, but are not limited to AmpliTaq DNA polymerase and AmpliTaq Gold DNA polymerase (Thermo Fisher Scientific).

It is to be understood, however, that DNA polymerases from other organisms can also be used herein without departing from the scope or preferred embodiments thereof. As an alternative to isolation, DNA polymerases are available commercially from, for example, Life Technologies (Carlsbad, CA), New England BioLabs (Beverly, MA), Finnzymes Oy (Espoo, Finland), Stratagene (La Jolla, CA), Boehringer Mannheim Biochemicals (Indianapolis, IN) and Perkin Elmer Cetus (Norwalk CT). It is to be understood that a variety of DNA polymerases can be used in the present compositions, methods and kits, including polymerases not specifically disclosed herein, without departing from the scope or preferred embodiments thereof.

In certain embodiments, the concentration of the thermostable DNA polymerase in the composition ad reaction mixtures disclosed herein is about 0.01 to about 500 units per microliter, about 0.01 to about 50 units per microliter, about 0.01 to about 25 units per microliter, about 0.01 to about 10 units per microliter, about 0.1 to about 5 units per microliter, about 0.1 to about 2 units per microliter, about 0.1 to about 1 unit per microliter or about 0.1 to about 0.5 units per microliter (units per microliter = U/µL), including all concentrations and concentration ranges within any of the forgoing.

In some embodiments, the compositions provided herein comprise an antifoam agent. An antifoam agent, as used herein, is a surface acting chemical which acts to facilitate gas release and to counteract foaming caused, for example, by mixing a reaction. Antifoam agents may prevent, eliminate, and/or reduce foam. Antifoam agents may additionally impart positive ancillary surface properties, such as wetting, dispersion, emulsification, and solubilization. In some embodiments, the antifoam agent of the composition provided is other than a detergent. In some embodiments, a combination of different antifoam agents may be present in the compositions described herein. In some embodiments, the antifoam agent or agents is/are present in an amount effective to enhance sensitivity of a PCR reaction.

Many chemical compounds can be used as antifoam agents, including without limitation, alkyl polyoxyalkylene glycol ethers; esters; alcohols; siloxanes; silicons; sulfites; sulfonates; fatty acids and their derivatives. A variety of such agents are known and commercially available from sources including J. T. Baker, Spectrum Chemicals, Dow Corning Corporation, and Sigma-Aldrich Company. Antifoam compositions may comprise a single component or multiple components which may be combined by simply mixing together. In some embodiments, antifoam agents for use in the provided compositions include, without limitation, siloxane polymers, mixtures of organic non-silicone polypropylene based polyether dispersions, silicone emulsions, non-ionic simethicone emulsions, organic fatty acid ester-type antifoams. For example, organosiloxanes, a well-known class of silicone-based antifoam agents, include pure silicon oils such as dimethylpolysiloxanes as well as polysiloxane/polyoxyalkylene block copolymers such as dimethylpolysiloxanes. In some embodiments, the antifoam agent is a silicon-based antifoam agent and is selected from XIAMETER^{®} AFE-1010, AFE-1430, AFE-1510, AFE-1520, AFE-2210, Antifoam A, Antifoam B, Antifoam C, Antifoam H-10, Antifoam SE-15, Antifoam SE-35, Antifoam SO-25, Antifoam Y-30, and Antifoam 289.

In some embodiments, the antifoam agent is a non-silicon-based antifoam agent. Examples of non-silicon-based antifoam agents include, without limitation, organic sulfonates, polyethers, organic phosphates, acetylenic glycols, fluorocarbons, polyalkene polyamines, and polyalkyleneimine compounds, including without limitation Antifoam 204 and Antifoam O-30.

In certain embodiments, the antifoam agent or agents is/are present in the compositions or reaction mixtures disclosed herein are at a concentration of about 0.0005% to about 0.5%, about 0.001% to about 0.1%, 0.002% to about 0.05%, or 0.005% to about 0.02%, or about 0.01% to about 0.05%, including all concentrations and concentration ranges within any of the forgoing.

In some embodiments, the compositions provided herein comprise at least one deoxynucleoside triphosphates (dNTP). In certain embodiments, the composition may further comprise a combination of one or more deoxyribonucleoside triphosphates (dNTPs) and one or more dNTP derivatives. In some embodiments, the composition comprises two to eight different dNTPs and/or dNTP derivatives. In some embodiments, the composition comprises two, three, four, five, or six different dNTPs and/or dNTP derivatives. Examples of dNTPs which can be included in the compositions, reaction mixtures or kits provided herein include, but are not limited to dATP, dCTP, dGTP, dTTP, dUTP, and/or dITP. Examples of possible dNTP derivatives include, but are not limited to 7-deaza-dGTP (such as 7-deaza-2-deoxy-dGTP), 7-deaza-dATP, alpha-thio-dATP, alpha-thio-dTTP, alpha-thio-dGTP, and/or alpha-thio-dCTP. In certain embodiments, the composition may further comprise a combination of one or more deoxyribonucleoside triphosphates (dNTPs) and one or more dNTP derivatives. In certain embodiments, the composition may further comprise one or more dideoxyribonucleoside triphosphates (ddNTPs) and/or one or more ddNTP derivatives. dNTPs, ddNTPs, and derivatives of each thereof, are available commercially from sources including Thermo Fisher Scientific, New England Biolabs, and Sigma-Aldrich Company. Such dNTPs, ddNTPs, and derivatives of each thereof may be unlabeled, or they may be detectably labeled by coupling them by methods known in the art with radioisotopes (e.g., ³H, ¹⁴C, ³²P or ³⁵S), vitamins (e.g., biotin), fluorescent moieties (e.g., fluorescein, rhodamine, Texas Red, or phycoerythrin), chemiluminescent labels, dioxigenin (DIG) and the like. Labeled dNTPs, ddNTPs, and derivatives of each thereof may also be obtained commercially, for example from Life Technologies (Carlsbad, CA) or Sigma Chemical Company (Saint Louis, MO).

The concentration of individual dNTPs,ddNTP, and/or derivatives of each thereof in the composition need not be identical. In some embodiments of the present compositions, dNTPs and/or ddNTPs can be added to give a concentration of each dNTP and/or ddNTP of about .001 mM to about 100 mM, about 0.01 mM to about 10 mM, about 0.1 mM to about 1 mM, or preferably about 0.2 mM to about 0.8 mM, including any concentrations or range of concentrations within any of the forgoing. In certain embodiments, concentrations of each dNTP and/or ddNTP in the composition is such so that its final concentration in the assembled PCR is about 0.015 mM to about 5 mM, about 0.05 to about 2 mM, about 0.1 mM to about 1 mM, about 0.1 mM to about 0.5 mM, about 0.15 mM to about 0.65 mM, about 0.15 mM to about 0.35 mM, or about 0.35 mM to about 0.65 mM, including any concentrations or range of concentrations within any of the forgoing. In some embodiments of the present compositions, dNTP derivatives and/or ddNTP derivatives can be added to give a concentration of each dNTP derivative and/or ddNTP derivative of about .001 mM to about 100 mM, about 0.01 mM to about 10 mM, about 0.1 mM to about 1 mM, or preferably about 0.2 mM to about 0.8 mM, including any concentrations or range of concentrations within any of the forgoing. In certain embodiments, concentrations of each dNTP and/or ddNTP in the composition is such so that its final concentration in the assembled PCR is about 0.015 mM to about 5 mM, about 0.05 to about 2 mM, about 0.1 mM to about 1 mM, about 0.1 mM to about 0.5 mM, about 0.15 mM to about 0.65 mM, about 0.15 mM to about 0.35 mM, or about 0.35 mM to about 0.65 mM, including any concentrations or range of concentrations within any of the forgoing.

In certain embodiments, the composition comprises a combination of a dNTP and a corresponding derivative of the same nucleotide thereof, such as a combination of a dNTP and the corresponding alpha-thio-dNTP derivative of the same nucleotide or a combination of a dNTP and the corresponding deaza-dNTP derivative of the same nucleotide. For example, in an embodiment, the composition provided comprises both dGTP and alpha-thio-dGTP. In another embodiment, the composition provided comprises both dGTP and 7-deaza-dGTP. In yet another embodiment, the composition comprises a combination of dATP and 7-deaza-dATP.

When both a dNTP and a derivative thereof are present in the composition, the relative concentration or concentration ratio of dNTP to derivative may vary. For example, in some embodiments, the relative concentration of dNTP:dNTP derivative is 1:1. In some embodiments, the dNTP concentration:dNTP derivative concentration is from about 100:1 to about 1:1, from about 50:1 to about 1.2:1, from about 25:1 to about 1.5:1, from about 10:1 to about 2:1. In other embodiments, the dNTP concentration:dNTP derivative concentration is from about 1:1 to about 1:100,from about 1:1.2 to about 1:50, from about 1:25 to about 1:1.5, from about 1:110 to about 1:2. In some embodiments, the dNTP concentration:dNTP derivative concentration is from about 2:1 to about 1:2. In some embodiments, the dNTP concentration:dNTP derivative concentration is from about 10:1 to about 2:1. In some embodiments, the dNTP concentration:dNTP derivative concentration is from about 1:2 to about 1:10. For example, in certain embodiments, the dGTP concentration:alpha-thio-dGTP concentration is from about 100:1 to about 1.5:1 or from about 50:1 to about 12:1. In other embodiments, the dGTP concentration:alpha-thio-dGTP concentration is from about 1:1.5 to about 1:100 or from about 1:12 to about 1:50. In some embodiments, the dGTP concentration:alpha-thio-dGTP concentration is from about 2:1 to about 1:2. In some embodiments, the dGTP concentration:alpha-thio-dGTP concentration is from about 10:1 to about 2:1. In some embodiments, the dGTP concentration:alpha-thio-dGTP concentration is from about 1:2 to about 1:10. In certain embodiments, the dGTP concentration:7-deaza-dGTP concentration is from about 100:1 to about 1.5:1 or from about 50:1 to about 12:1. In other embodiments, the dGTP concentration:7-deaza-dGTP concentration is from about 1:1.5 to about 1:100 or from about 1:12 to about 1:50. In some embodiments, the dGTP concentration: 7-deaza-dGTP concentration is from about 2:1 to about 1:2. In some embodiments, the dGTP concentration: 7-deaza-dGTP concentration is from about 10:1 to about 2:1. In some embodiments, the dGTP concentration: 7-deaza-dGTP concentration is from about 1:2 to about 1:10.

In some embodiments, provided herein is an assembled PCR composition comprising a thermostable DNA polymerase, a magnesium salt, an antifoam agent, and a combination of dATP, dCTP, dUTP, and 7-deaza-dGTP. In an embodiment, provided herein is an assembled PCR composition comprising a thermostable DNA polymerase, a magnesium salt, an antifoam agent, and a combination of dCTP, dGTP, dUTP, and 7-deaza-dATP. In an embodiment, provided herein is an assembled PCR composition comprising a thermostable DNA polymerase, a potassium salt, an antifoam agent, and a combination of dGTP and 7-deaza-dGTP. In an embodiment, provided herein is an assembled PCR composition comprising a thermostable DNA polymerase, a potassium salt, an antifoam agent, and a combination of dATP and 7-deaza-dATP. In an embodiment, provided herein is an assembled PCR composition comprising a thermostable DNA polymerase, a magnesium salt, an antifoam agent, and a combination of dGTP and 7-deaza-dGTP. In an embodiment, provided herein is an assembled PCR composition comprising a thermostable DNA polymerase, a magnesium salt, an antifoam agent, and a combination of dATP and 7-deaza-dATP. In certain embodiments, the antifoam agent is a silicone-based antifoam agent. In one embodiment, the silicone-based antifoam agent is selected from XIAMETER^{®} AFE-1010, AFE-1430, AFE-1510, AFE-1520, AFE-2210, Antifoam A, Antifoam B, Antifoam C, Antifoam H-10, Antifoam SE-15, Antifoam SE-35, Antifoam SO-25, Antifoam Y-30, and Antifoam 289. In other embodiments, the antifoam agent is a non-silicone-based antifoam agent. In one embodiment, the non-silicone-based antifoam agent is selected from organic sulfonates, polyethers, organic phosphates, acetylenic glycols, fluorocarbons, polyalkene polyamines, and polyalkyleneimine compounds, including without limitation Antifoam 204 and Antifoam O-30.

The compositions provided may also comprise one or more PCR inhibitor blocking agents. Such PCR inhibitor blocking agents can be added to the compositions or reaction mixtures disclosed herein to assist in overcoming the inhibition of PCR reactions by a variety of compounds often found in biological samples used for nucleic acid preparation, isolation, or purification. In some embodiments, the PCR inhibitor blocking agent(s) can reduce the amount of PCR inhibition by a PCR inhibitor or inhibitors from some percentage above zero up to100% compared to the level of inhibition observed in the absence of such PCR inhibitor blocking agents. For example, inhibition can be reduced by at least about 0.5%, about 1%, about 2%, about 5%, about 10%, about 20%, about 50%, about 75%, about 90%, about 95% or about 100% or any percentage in between.

In some embodiments, the PCR inhibitor blocking agent as described herein is a protein. In some embodiments, such proteins can include, but are not limited to, albumins, gelatins, and DNA-binding proteins, or peptide or polypeptide variants, fragments or derivatives thereof. In some embodiments, other non-protein based PCR inhibitor blocking agents for use in the present teachings can include, for example, deferoxamine mesylate. In some embodiments, the compositions can comprise a combination of PCR inhibitor blocking agents and/or proteins. For example, in some embodiments the compositions comprise an albumin, a gelatin, or a combination of albumin and gelatin. In some embodiments, the albumin or gelatin may be selected from serum albumin, fish gelatin, or a combination of serum albumin and fish gelatin. The serum albumin can be from any animal, e.g., bovine serum albumin (BSA), human serum albumin (HSA). In some embodiments, the albumin is derived from other species of animals which are well-known to those of skill in the art. In some embodiments the albumin is a recombinant albumin, such as recombinant BSA (rBSA). The gelatin can be from any animal, e.g., fish gelatin, bovine gelatin. In some embodiments, the gelatin is derived from other species of animals which are well-known to those of skill in the art. In some embodiments the gelatin is a recombinant gelatin, such as recombinant human gelatin. In some embodiments, the DNA-binding proteins can include, but are not limited to T4 gene 32 protein (T4 gp32).

Certain PCR inhibitor blocking compounds or agents can be added to the present compositions to give a concentration in the composition of about .0001 mg/mL to about 10 mg/mL, about .001 mg/mL to about 8 mg/mL, about 0.01 mg/mL to about 6 mg/mL, about 0.05 mg/mL to about 4 mg/mL, about 0.1 mg/mL to about 3 mg/mL or about 0.5 mg/mL to about 2 mg/mL, including any concentrations or range of concentrations within any of the forgoing. Certain PCR inhibitor blocking agents can also be added as a percentage of the final concentration of the composition, for example, from about 0.001% to about 15%, about 0.05% to about 10%, about 0.01% to about 5%, or about 0.1% to about 1%, including any concentrations or range of concentrations within any of the forgoing. In some embodiments, the compositions as described herein comprise a PCR inhibitor blocking protein, such as albumin, at a concentration such that its concentration in an assembled PCR is about 0.001 mg/mL to about 10.0 mg/mL, 0.005 mg/mL to about 5.0 mg/mL, about 0.01 mg/mL to about 4.0 mg/mL, about 0.05 mg/mL to about 3.0 mg/mL or about 0.1 mg/mL to about 2.0 mg/mL, including any concentrations or range of concentrations within any of the forgoing. In another embodiment, the compositions as described herein comprise a PCR inhibitor blocking protein, such as gelatin at a concentration such that its concentration in an assembled PCR is about 0.005% (w/v) to about 2% (w/v), about 0.01% (w/v) to about 1.0% (w/v), and more specifically about 0.05% (w/v) to about 0.5% (w/v), including any concentrations or range of concentrations within any of the forgoing. In yet another embodiment, the compositions as described herein comprise a combination of albumin and gelatin at any of the foregoing concentrations. In some preferred embodiments, the albumin is at about 0.05mg/mL to 5 mg/mL and gelatin is at a concentration of about 0.01% (w/v) to about 1%. In some embodiments, the albumin is bovine serum albumin (BSA), and the gelatin is a fish gelatin and/or a bovine gelatin.

In some embodiments, the composition can comprise one, two, three, four, five or more different PCR inhibitor blocking proteins and/or agents. For example, in some embodiments the composition comprises an albumin, a fish gelatin and a bovine gelatin. In some embodiments, the concentration of each PCR inhibitor blocking protein and/or agent is the same. In some embodiments the concentration of each PCR inhibitor blocking protein and/or agent is different. In some embodiments, one or more PCR inhibitor blocking agents are added to the compositions or reaction mixtures to help to overcome inhibition of PCR by a variety of inhibitors often found in biological samples, Such inhibitors include, for example, heparin (blood); hematin (blood); EDTA (blood); citrate (blood); immunoglobin G (blood, serum); humic acid (soil, feces); lactoferrin (milk, saliva, other secretory fluids); urea (urine); plant polysaccharides (plants); melanin (skin, hair); myoglobin (tissue); and indigo dye (textiles). The addition of PCR inhibitor blocking agents, both individually and in combination, can increase tolerance to such PCR inhibitor contaminants. Thus, the present compositions can further comprise agents that work alone or in combination to increase tolerance to various PCR inhibitors including, for example, humic acid, hematin, and heparin. In some embodiments each PCR inhibitor blocking agent or protein in the composition or reaction mixture can block a different inhibitor or group of inhibitors than the other PCR inhibitor blocking agent or protein in the composition or reaction mixture. In some embodiments, the different PCR inhibitor blocking agents or proteins in the composition or reaction mixture can block the same inhibitor or group of inhibitors. In some embodiments, the different PCR inhibitor blocking agents or proteins in the composition or reaction mixture can block an overlapping number of inhibitor of a group of inhibitors. For example, in some embodiments, gelatin is effective at reducing PCR inhibition by at least humic acid and heparin, and albumin is effective at reducing PCR inhibition by at least humic acid and hematin.

According to the present teachings, the compositions provided herein can comprise one or more primers which facilitate the synthesis of a DNA molecule (e.g., a single-stranded cDNA molecule or a double-stranded cDNA molecule) complementary to all or a portion of nucleic acid template (RNA or DNA). Additionally, these primers can be used in amplifying nucleic acid molecules in accordance with the present teachings. Oligonucleotide primers can be any oligonucleotide of two or more (e.g., 2, 3, 4, 5, 8, 10, 15, 20, 25, and so on) nucleotides in length. Such primers include, but are not limited to, target-specific primers (which are preferably gene-specific primers), oligo(dT) primers, random primers or arbitrary primers. Additional primers that can be used for amplification of the DNA molecules according to the methods disclosed herein will be apparent to one of ordinary skill in the art. It is to be understood that a vast array of primers can be useful in the present compositions, methods and kits, including those not specifically disclosed herein, without departing from the scope or preferred embodiments thereof.

In some embodiments, the final concentration of primers can range from about 25 nM to about 2000 nM, such as about 50 nM to about 1700 nM, about 75 nM to about 1500 nM, about 100 nM to about 1200 nM, about 200 nM to about 1000 nM, or any range in between. In some exemplary embodiments, the concentration of each primer is between about 400 nM to about 900 nM, including all amounts or ranges in between.

In accordance with the present teachings, the compositions can further comprise probes for the detection of target nucleic acids. Various probes are known in the art, for example (TaqMan^{™} probes (see, e.g., U.S. Pat. No. 5,538,848), various stem-loop molecular beacons (see, e.g., U.S. Pat. Nos. 6,103,476 and 5,925,517 and Tyagi and Kramer, 1996, Nature Biotechnology 14:303-308), stemless or linear beacons (see, e.g., WO 99/21881), PNA Molecular Beacons^{™} (see, e.g., U.S. Pat. Nos. 6,355,421 and 6,593,091), linear PNA beacons (see, e.g., Kubista et al., 2001, SPIE 4264:53-58), non-FRET probes (see, e.g., U.S. Pat. No. 6,150,097), Sunrise^{™}/Amplifluor^{™} probes (U.S. Pat. No. 6,548,250), stem-loop and duplex Scorpion^{™} probes (see, e.g., Solinas et al., 2001, Nucleic Acids Research 29:E96 and U.S. Pat.No. 6,589,743), bulge loop probes (see, e.g., U.S. Pat. No. 6,590,091), pseudo knot probes (see, e.g., U.S. Pat. No. 6,589,250), cyclicons (see, e.g., U.S. Pat. No. 6,383,752), MGB Eclipse^{™} probe (Epoch Biosciences), hairpin probes (see, e.g., U.S. Pat. No. 6,596,490), peptide nucleic acid (PNA) light-up probes, self-assembled nanoparticle probes, and ferrocene-modified probes described, for example, in U.S. Pat. No. 6,485,901; Mhlanga et al., 2001, Methods 25:463-471; Whitcombe et al., 1999, Nature Biotechnology. 17:804-807; Isacsson et al., 2000, Molecular Cell Probes. 14:321-328; Svanvik et al., 2000, Anal Biochem. 281:26-35; Wolffs et al., 2001, Biotechniques 766:769-771; Tsourkas et al., 2002, Nucleic Acids Res. 30:4208-4215; Riccelli et al., 2002, Nucleic Acids Res. 30:4088-4093; Zhang et al., 2002 Shanghai. 34:329-332; Maxwell et al., 2002, J. Am. Chem. Soc. 124:9606-9612; Broude et al., 2002, Trends Biotechnol. 20:249-56; Huang et al., 2002, Chem Res. Toxicol. 15:118-126; and Yu et al., 2001, J. Am. Chem. Soc 14:11155-11161. Probes can comprise reporter dyes such as, for example, 6-carboxyfluorescein (6-FAM) or tetrachlorofluorescin (TET). Detector probes can also comprise quencher moieties such as tetramethylrhodamine (TAMRA), Black Hole Quenchers (Biosearch), Iowa Black (IDT), QSY quencher (Thermo Fisher Scientific), and Dabsyl and Dabcel sulfonate/carboxylate Quenchers (Epoch). Probes can also comprise two probes, wherein for example a fluor is on one probe, and a quencher on the other, wherein hybridization of the two probes together on a target quenches the signal, or wherein hybridization on a target alters the signal signature via a change in fluorescence.

Exemplary detectable labels include, for instance, a fluorescent dye or fluorphore (e.g., a chemical group that can be excited by light to emit fluorescence or phosphorescence), "acceptor dyes" capable of quenching a fluorescent signal from a fluorescent donor dye, and the like. Suitable detectable labels may include, for example, fluoresceins (e.g., 5-carboxy-2,7-dichlorofluorescein; 5-Carboxyfluorescein (5-FAM); 5-HAT (Hydroxy Tryptamine); 6-HAT; 6-JOE; 6-carboxyfluorescein (6-FAM); FITC); Alexa fluors (e.g., 350, 405, 430, 488, 500, 514, 532, 546, 555, 568, 594, 610, 633, 635, 647, 660, 680, 700, 750); BODIPY^{®} fluorophores (e.g., 492/515, 493/503, 500/510, 505/515, 530/550, 542/563, 558/568, 564/570, 576/589, 581/591, 630/650-X, 650/665-X, 665/676, FL, FL ATP, FI-Ceramide, R6G SE, TMR, TMR-X conjugate, TMR-X, SE, TR, TR ATP, TR-X SE), coumarins (e.g., 7-amino-4-methylcoumarin, AMC, AMCA, AMCA-S, AMCA-X, ABQ, CPM methylcoumarin, coumarin phalloidin, hydroxycoumarin, CMFDA, methoxycoumarin), calcein, calcein AM, calcein blue, calcium dyes (e.g., calcium crimson, calcium green, calcium orange, calcofluor white), Cascade Blue, Cascade Yellow; Cy^{™} dyes (e.g., 3, 3.18, 3.5, 5, 5.18, 5.5, 7), cyan GFP, cyclic AMP Fluorosensor (FiCRhR), fluorescent proteins (e.g., green fluorescent protein (e.g., GFP. EGFP), blue fluorescent protein (e.g., BFP, EBFP, EBFP2, Azurite, mKalama1), cyan fluorescent protein (e.g., ECFP, Cerulean, CyPet), yellow fluorescent protein (e.g., YFP, Citrine, Venus, YPet), FRET donor/acceptor pairs (e.g., fluorescein/tetramethylrhodamine, IAEDANS/fluorescein, EDANS/dabcyl, fluorescein/fluorescein, BODIPY^{®} FL/BODIPY^{®} FL, Fluorescein/QSY7 and QSY9), LysoTracker and LysoSensor (e.g., LysoTracker Blue DND-22, LysoTracker Blue-White DPX, LysoTracker Yellow HCK-123, LysoTracker Green DND-26, LysoTracker Red DND-99, LysoSensor Blue DND-167, LysoSensor Green DND-189, LysoSensor Green DND-153, LysoSensor Yellow/Blue DND-160, LysoSensor Yellow/Blue 10,000 MW dextran), Oregon Green (e.g., 488, 488-X, 500, 514); rhodamines (e.g., 110, 123, B, B 200, BB, BG, B extra, 5-carboxytetramethylrhodamine (5-TAMRA), 5 GLD, 6-Carboxyrhodamine 6G, Lissamine, Lissamine Rhodamine B, Phallicidine, Phalloidine, Red, Rhod-2, 5-ROX (carboxy-X-rhodamine), Sulphorhodamine B can C, Sulphorhodamine G Extra, Tetramethylrhodamine (TRITC), WT), Texas Red, Texas Red-X, VIC and other labels described in, e.g., US Publication No. 2009/0197254), among others as would be known to those of skill in the art.

In some embodiments, the probes are designed according to the methods and principles described in, for example, U.S. Patent No. 6,727,356 (the disclosure of which is incorporated herein by reference in its entirety). Some probes can be sequence-based, for example 5' nuclease probes and some, such as SYBR^{®} Green can be non-sequence specific DNA-binding dyes. In some preferred embodiments, the detector probe is a TaqMan^{™} probe (Applied Biosystems, Foster City, CA). It is to be understood that a wide variety of probes are known in the art that can be used in the present compositions, methods and kits, including those not specifically disclosed herein.

In some embodiments, the probe concentration in a working solution can range from about 5 nM to about 750 nM, such as about 10 nM to about 600 nM, about 25 nM to about 500 nM, about 50 nM to about 400 nM, about 75 nM to about 300 nM, or any number in between. In some exemplary embodiments, the concentration of each probe is between about 100 nM to about 250 nM, including all amounts or ranges in between.

In accordance with the present teachings, one or more additional components and/or additives can be incorporated in the present compositions, methods, and kits to optimize the synthesis of nucleic acids from a nucleic acid template. In some embodiments, the composition can comprise additional components and/or additives which are capable of facilitating or enhancing nucleic acid synthesis reactions (e.g., reagents for facilitating or enhancing PCR). Components and/or additives which enhance nucleic acid synthesis can be organic or inorganic compounds. Some components and/or additives useful in the present compositions, methods, and kits include polypeptides as well as nonpolypeptide components. Such components and/or additives can include, but are not limited to, for example, single-stranded binding DNA-binding (SSB) proteins, sulfur-containing compounds, acetate-containing compounds, dimethylsulfoxide (DMSO), glycerol, formamide, betaine, tetramethylammonium chloride (TMAC), ectoine, sodium azide, kathon, polyols, NaN₃, buffers, surfactants, detergents (e.g., TWEEN 20, NP-40, Tritin X-100, and CHAPS), a component or compound used for hot start PCR, a passive reference control to minimize sample-to-sample and/or well-to-well variations in quantitative real-time DNA-detection assays, and/or uracil DNA glycosylase, to name just a few. The composition may further comprise crowding agents such as Ficoll 70, glycogen, and polyethylene glycol (PEG). Those of ordinary skill in the art will be able to identify additional reagents and/or additives for use in accordance with the present compositions, methods and kits.

In some aspects, the compositions or kits provided herein can comprise glycerol. In some embodiments, glycerol is present in the composition at a concentration such that its concentration is about 5 to about 50% (w/v), or from about 10% (w/v) to about 30% (w/v), including all concentrations or range of concentrations in between any of the foregoing amounts.

In some aspects, the compositions or kits provided herein can comprise NaN₃. In some embodiments, NaN₃ is present in the composition at a concentration such that its concentration is about 0.005% (w/v) to about 0.05% (w/v), or from about 0.01% (w/v) to about 0.1% (w/v).

In some aspects, the compositions or kits provided herein can comprise a detergent or combination of detergents. In some embodiments, the detergent(s) is/are present in an amount effective to enhance sensitivity of a PCR reaction. The detergent(s) can be an anionic detergent, a cationic detergent, a zwitterionic detergent, and/or a nonionic detergent. In some embodiments, the nonionic detergent(s) can be, without limitation, TRITON X-100^{®}, Nonidet P-40 (NP-40), TWEEN 20, TWEEN 80, Brij 30, Brij 35, and/or Brij 58. In some embodiments, the composition comprises any nonionic detergent, such as TWEEN 20. In some embodiments, the composition comprises a nonionic detergent other than TWEEN 20. In some embodiments, the composition does not contain and detectable TWEEN 20. In some embodiments, the composition provided herein comprises a nonionic detergent other than TWEEN 20 present in an amount effective to enhance sensitivity of a PCR reaction. In some embodiments, the composition comprises TRITON X-100^{®}. In some embodiments, the composition comprises NP-40. In some embodiments, the composition comprises Brij 30, Brij 35, or Brij 58. In some embodiments, the composition comprises any combination of the aforementioned detergents. The detergent can be present in the composition at a concentration that is about 0.001% to about 0.50% (w/v), about 0.005 to about 0.1% (w/v), or about 0.01% (w/v) to about 0.05% (w/v), including all concentrations or range of concentrations in between any of the foregoing amounts.

In some aspects, the compositions, reaction mixtures, or kits provided herein can comprise a buffer component, such as a buffered salt solution. In some embodiments, the buffer agent provides appropriate pH conditions to maintain stability of the DNA polymerase enzyme. The terms "stable" and "stability" as used herein generally mean the retention by a composition, such as an enzyme composition, of at least 70%, preferably at least 80%, and most preferably at least 90%, of the original enzymatic activity (in units) after the enzyme or composition containing the enzyme has been stored for about 3 days at a temperature of about room temperature (e.g., about 20°C to about 25°C), about one to eight weeks at a temperature of about 4°C, about two to six months at a temperature of about -20°C, and about six months or longer at a temperature of about -80 °C. Examples of such buffering agents can include, for example, TRIS, TRICINE, BIS-TRICINE, HEPES, MOPS, TES TAPS, PIPES, and CAPS. In some embodiments, the buffer concentration of the compositions and/or reaction mixtures as disclosed herein is between about 1 mM and about 500 mM, between about 5 mM and about 250 mM, between about 10 mM and about 200 mM, between about 25 mM and about 150 mM, and between about 50 mM and about 100 mM, including any concentration or range falling within the forgoing amounts. It is to be understood that a wide variety of buffers (or buffer salts) are known in the art that, including those not specifically disclosed herein which can be used in accordance with the present compositions, methods and kits.

Examples of salts suitable for inclusion in the compositions provided herein include, without limitation, potassium chloride, potassium acetate, potassium sulfate, ammonium sulfate, ammonium chloride, ammonium acetate, magnesium chloride, magnesium acetate, magnesium sulfate, manganese chloride, manganese acetate, manganese sulfate, sodium chloride, sodium acetate, lithium chloride and lithium acetate. In certain embodiments, the composition comprises more than one (e.g., two, three, four, five, six, etc.) salt component. For example, the compositions described herein can comprise two different salts. In other embodiments, the compositions comprise three different salts. In still other embodiments, the compositions comprise four different salts. In one embodiment, the composition comprises a particular salt at a concentration of about 0.5 mM to about 1000 mM, about 1 mM to about 500 mM, about 5 mM to about 250 mM, about 10 mM to about 100 mM, about 5 mM to about 50 mM, and about 2 mM to about 10 mM, including any concentration or range falling within the forgoing ranges. Without limitation, in certain embodiments where the composition comprises a salt such as potassium chloride, potassium acetate, potassium sulfate, ammonium sulfate, ammonium chloride, and/or ammonium acetate, the composition comprises the salt at a concentration such that in an assembled PCR is about 5 mM to about 250 mM, 5 mM to about 150 mM, about 10 mM to about 120 mM, about 20 mM to about 100 mM, about 30 mM to about 90 mM, or about 40 to about 80 mM, including any concentration falling within the forgoing ranges. Without limitation, in certain embodiments where the composition comprises a salt such as magnesium acetate, magnesium sulfate, manganese chloride, manganese acetate, or manganese sulfate, the composition comprises the salt at a concentration such that in an assembled PCR is about 0.5 mM to about 100 mM, about 1 mM to about 75 mM, about 1.5 mM to about 50 mM, about 2 mM to about 30 mM, about 3 mM to about 15 mM, about 4 mM to about 10 mM, or about 1 mM to about 5 mM, including any concentration falling within the forgoing ranges. It is to be understood that a wide variety of salts or salt solutions are known in the art that can be used in accordance with the present compositions, methods and kits, including those not specifically disclosed herein.

In some aspects, the compositions or kits provided herein can comprise a passive reference control component. In some embodiments, the passive reference control minimizes sample-to-sample and/or well-to-well variations in quantitative real-time nucleic acid-detection assays and/or can be included at a concentration allowing its use as detectable control. In an embodiment, a reference chromophore, specifically a fluorophore, is included as the passive reference control. In an embodiment, the reference chromophore is the fluorescent dye. In some embodiments, the fluorescent dye is a ROX dye (Thermo Fisher Scientific). In some embodiments, the fluorescent dye is a MUSTANG PURPLE dye (Thermo Fisher Scientific). The passive reference can be included in the composition at a concentration such that its concentration in the assembled PCR is about 10 to about 750 nM or about 20 nM to about 500 nM, or about 50 nM to about 200 nM, including any concentration falling within the forgoing ranges.

In some aspects, uracil DNA glycosylase (UNG or UDG) can be included in the compositions or kits provided herein. The enzyme is commercially available from a number of commercial sources, for example Thermo Fisher Scientific, Enzymatics, New England Biolabs, Genscript, or USB. In some embodiments, UNG is thermolabile. In other embodiments, UNG is thermostable. Thermolabile or thermostable UNG can be included in the composition at a concentration such that its concentration in the final assembled PCR is about 0.0001 U/µL to about 50 U/µL, about 0.0005 U/µL to about 10 U/µL, about 0.001 U/µL to about 5 U/µL, 0.005 U/µL to about 1.0 U/µL, or 0.01 U/µL to about 0.5 U/µL (U/µL = units per microliter), including any concentration or range falling within the forgoing ranges.

Undesired amplification reactions, which can occur during the PCR process, usually begin during assembly of the reaction mixtures, or while the thermal cycler is heating to the initial denaturation temperature. These spurious reactions can be minimized by performing "hot start" amplification. In general, hot start techniques limit the availability of an essential reaction component until an elevated temperature, often >60°C, is reached.

The compositions, kits, and methods in accordance with the present teachings can include "hot start" mechanisms, components or steps, as a means to further prevent, reduce or eliminate nonspecific nucleic acid synthesis. Several methods exist for performing hot start reactions including manual techniques, barriers, reversible polymerase inactivation, and specially-designed hairpin primers. In some embodiments, components or compounds used for hot start reactions, such as in PCR, can be any of those which prevent non-specific amplification of DNA by inactivating polymerase activity at lower temperature, such as during the annealing phase, while allowing reactivation or activation of the polymerase activity at a higher temperature, such as during the extension phase. Such examples of hot start components can include, but are not limited to, for example, an antibody, a chemical modification (e.g., of the polymerase), an oligonucleotide, an aptamer, a specially-designed primer, a binding protein, and/or a sequestration wax bead. Wax beads for hot start PCR are commercially available, e.g., HotStart ^{™} Storage reaction tubes (Thermo Fisher Scientific). Selection of a suitable hot start aptamer can be performed by a method known in the art or a commercially available hot start aptamer can be used. Similarly, selection of a suitable hot start hairpin primer can be performed by a method known in the art or a commercially available hot start primer can be used. Antibodies for hot start PCR can be generated or selected by various methods known in the art. Alternatively, a commercially available antibody can be used, for example, the TaqStart Antibody (Clontech) which is effective with any Taq-derived DNA polymerase, including native, recombinant, and N-terminal deletion mutants. In some cases a suitable hot start primer can be a primer specially designed to have secondary structure (such as a hairpin primer) which prevents the primer from annealing until cycling temperatures cause them to denature and unfold. An appropriate concentration of the compound or reagent for hot start PCR in the assembled reaction mixtures can be determined by a number of methods known in the art or, for a commercial product, suggested by the manufacturer. Some of the hot start mechanisms, components or steps are described in more detail below.

In general, manual hot start methods usually, though not always, require the researcher to withhold a critical component, usually magnesium or the polymerase, until the reaction has been heated. The withheld component then is added to initiate the reaction. A second method uses a physical barrier (e.g., wax) to separate a critical component from the template and primers. U.S. Pat. No. 5,565,339 describes using a wax barrier to separate the various PCR reagents from each other in a test tube. U.S. Pat. No. 5,413,924 describes using a paraffin wax bead to sequester the DNA polymerase.

An alternative method of hot start amplification is reversible polymerase inactivation. The polymerase is reacted with an antibody or an oligonucleotide aptamer that binds to the polymerase's nucleotide binding domain, rendering the polymerase inactive. For example, a monoclonal antibody to Taq polymerase, such as the anti-Taq DNA polymerase antibody available from Sigma, is introduced into the reaction mixture. Upon heating, the compound dissociates from the polymerase, restoring enzyme activity. In another example, U.S. Pat. No. 5,677,152 describes a method in which the DNA polymerase is chemically modified to ensure that it only becomes active at elevated temperatures.

Another approach to achieve hot start amplification is to design primers that will self-anneal to form specific hairpin structures. The hairpin primers will not be able to anneal to the target nucleic acid while in the hairpin conformation. The hairpin primers will remain in a hairpin conformation until heated to a denaturation temperature. However, if the hairpin structure includes a single-strand extension, then the hairpin structure itself resembles a primer annealed to a template and can result in strand extension.

Various other hot start components or mechanisms, in addition to those described above, are also well known to those of ordinary skill in the art and will be readily selectable based on their ability to work in accordance with the present teachings. In certain embodiments, compositions, reaction mixtures, kits, and methods are provided that comprise at least two different hot start mechanisms, components, or steps that are used to inhibit or substantially inhibit the polymerase activity of a nucleic acid polymerase under a first condition (such as at a lower temperature) and allow polymerase activation under a second condition (such as at a higher temperature). Such hot start mechanisms include, but are not limited to those described above, including antibodies or combinations of antibodies that block DNA polymerase activity at lower temperatures, oligonucleotides that block DNA polymerase activity at lower temperatures, reversible chemical modifications of the DNA polymerase that dissociate at elevated temperatures, amino acid modifications of the DNA polymerase that provide reduced activity at lower temperatures, fusion proteins that include hyperstable DNA binding domains and topoisomerase, temperature dependent ligands that inhibit the DNA polymerase, single stranded binding proteins that sequester primers at lower temperatures, modified primers or modified dNTPs.

In some embodiments, the compositions comprises a thermostable DNA polymerase, a salt, an antifoam agent, dNTPs, a combination of PCR inhibitor blocking agents, a buffer, a hot start component, glycerol, a nonionic detergent, and a passive reference dye. In other embodiments, the composition comprises a thermostable DNA polymerase, a potassium salt, a magnesium salt, a silicon-based antifoam agent, dNTPs including a dNTP/dNTP derivative combination, an albumin, fish gelatin, a buffer, a hot start component, glycerol, a nonionic detergent other than TWEEN 20, and a passive reference dye. The components may be substituted or modified as determined by those of skill in the art. It is to be understood that a wide variety of additional components known in the art can be useful in the present compositions, methods and kits, including those not specifically disclosed herein. Those of skill in the art will also understand the methods required to determine the particular conditions or concentrations for optimal use of each component in accordance with the present teachings.

In one aspect, the compositions can be provided as a concentrated stock solution or mix. As used herein, the term "concentrated stock" means at a concentration that requires further dilution in order to achieve optimal concentration for use in a solution to perform a particular function (such as PCR amplification). For example, compositions may be stock solutions of about 2X, about 3X, about 4X, about 5X, about 6X, about 10X, and so on, including any amount between any of the forgoing. In some embodiments, the compositions may require greater than 2X, greater than 3X, greater than 4X, greater than 5X, greater than 6X, greater than 10X, and so on, including any amount between any of the forgoing, dilution to be at working, or optimal, concentration for use, for example, in nucleic acid synthesis or amplification methods. In some embodiments, the compositions described herein may also be provided at a working concentration or as a "working solution or mix." As used herein "working concentration" or "working solution or mix" is used to refer to a solution or mix that is at or near the optimal concentration used to perform a particular function or reaction (such as amplification or PCR). In some embodiments, a composition that is at a working concentration needs no or minimal dilution prior to use. For example, in some embodiments a compositions that is at a working concentration is at about a 1X, about a 1.25X, about a 1.5X, about a 1.8X, about a 2X, or about a 2.2X concentration of the final concentration in the assembled reaction mixture.

In some embodiments, the compositions of the present teachings can be formulated as master mixes. Master mixes can improve efficiency and reduce errors associated with the assembly of large number of reactions required for high-throughput analysis. In some embodiments, master mixes can contain combination of reagents common to all reactions. For example, in some embodiments the master mix can contain a buffer, a salt, such as MgCl₂, deoxynucleoside triphosphates (dNTPs), a thermostable DNA polymerase, a detergent, and a PCR inhibitor blocking agent. For use in certain assays, each reaction would then contain an aliquot of the common master mix and a specific target nucleic acid template and at least one primer. In some embodiments, master mixes can be manufactured and distributed as a concentrated stock solution or mixture. The master mix can then be diluted when final reactions are assembled. In other embodiments, the master mix can be manufactured and distributed as a working solution or mixture.

In some aspects, the invention is also directed to compositions which are reaction mixtures. As used herein "reaction mixture" refers to a mixture of two or more substances which together can cause a reaction; or wherein a mixture of two or more substances can cause a chemical transformation or change. In some embodiments, the reaction mixtures comprise: (a) at least one polymerase; (b) at least one PCR inhibitor blocking agent; (c) at least one detergent; (d) at least one antifoam agent; (e) at least one salt; (f) a buffer; (g) at least one dNTP and/or dNTP derivative; (h) at least one primer; (i) and a nucleic acid sample. In some embodiments the reaction mixture can further comprise a labeled probe (e.g., TaqMan^{™} probe). In some embodiments, the polymerase of the reaction mixture is a thermostable DNA polymerase. In some embodiments, the thermostable DNA polymerase of the reaction mixture is Taq DNA polymerase. In some embodiments, the PCR inhibitor blocking agent of the reaction mixture is a protein. In some embodiments, the PCR inhibitor blocking protein of the reaction mixture is selected from albumin, gelatin, or a combination of albumin and gelatin. In some embodiments, the detergent of the reaction mixture is a non-ionic detergent. In some embodiments, the non-ionic detergent of the reaction mixture is a non-ionic detergent other than Tween-20. In some embodiments, the antifoam agent of the reaction mixture is a silicone-based agent. In some embodiments, the silicone-based agent of the reaction mixture is selected from XIAMETER^{®} AFE-1010, AFE-1430, AFE-1510, AFE-1520, AFE-2210, Antifoam A, Antifoam B, Antifoam C, Antifoam H-10, Antifoam SE-15, Antifoam SE-35, Antifoam SO-25, Antifoam Y-30, and Antifoam 289. In some other embodiments, the antifoam agent of the reaction mixture is a non-silicone-based agent. In some embodiments, the non-silicone-based agent of the reaction mixture is selected from organic sulfonates, polyethers, organic phosphates, acetylenic glycols, fluorocarbons, polyalkene polyamines, and polyalkyleneimine compounds, including without limitation Antifoam 204 and Antifoam O-30.

In some embodiments, the dNTP of the reaction mixture is selected from dGTP, dCTP, dATP and dTTP. In some embodiments, the dNTP derivative of the reaction mixture is selected from 7-deaza-dGTP (such as 7-deaza-2-deoxy-dGTP), 7-deaza-dATP, alpha-thio-dATP, alpha-thio-dTTP, alpha-thio-dGTP, and alpha-thio-dCTP. In some embodiments, the reaction mixture comprises a dNTP/dNTP derivative blend comprising some combination of the aforementioned dNTPs and dNTP derivatives. In some embodiments, the nucleic acid template of the reaction mixture is DNA. In some embodiments, the DNA in the reaction mixture is genomic DNA (gDNA) or complementary DNA (cDNA).

In some embodiments, the compositions described herein can be packaged in a suitable container capable of holding the composition and which will not significantly interact with components of the composition. The container can be one designed to permit easy dispensing of the dosage form by individuals or by a liquid handling instrument. The containers of composition can be further packaged into multi-pack units. In some embodiments, the multi-pack units may further contain additional containers comprising additives or other reagents to be added to the composition prior to use in a reaction mixture, such as in a PCR.

In some embodiments, the compositions described herein can be in a liquid form, such as in a hydrated solution. In other embodiments, the compositions described herein can be in a gel form. A "gel" as used herein is a composition which is not solid or frozen at -20°C. In yet other embodiments, the compositions described herein can be in a dehydrated or dried form, such as in a lyophilized composition.

Also disclosed herein are kits comprising the compositions or packaged containers comprising the compositions. In some embodiments, the compositions can be assembled into kits for use in nucleic acid synthesis or amplification reactions. The kits can further comprise additional reagents used in one or more assays used to synthesize, detect or quantify nucleic acids. In one embodiment, the composition may be present in a kit for use in the amplification of a nucleic acid molecule, such as in PCR.

In some embodiments, the kits comprise a carrier, such as a box, carton, tube or the like, having in close confinement therein, a container, such as a vial, tube, ampule, plate, bottle and the like. When more than one enzyme is included in a kit (for example, a DNA polymerase and a reverse transcriptase or a DNA polymerase and UNG), the enzymes may be in a single container as mixtures of two or more enzymes (e.g., 2, 3, 4, 5, etc.), or in separate containers. The kits of the invention may also comprise (in the same or separate containers), a salt solution, an antifoam agent, a suitable buffer, a dNTP and/or dNTP derivative mixture or blend, a probe and/or a primer. In some embodiments, the DNA polymerase, salt, antifoam agent, dNTP and/or dNTP derivative mixtures or blends, and buffer are combined in a single tube or container.

In another aspect, the kits may comprise master mix compositions for use in nucleic acid synthesis or amplification reactions. Such compositions may be formulated as concentrated stock (e.g., 2X, 3X, 4X, 5X, 6X, etc.). In some embodiments, the compositions can be formulated as concentrated stock in a single tube or container, comprising a DNA polymerase, a salt, an antifoam agent, and dNTPs. In some embodiments, such concentrated stock compositions may further comprise a combination of PCR inhibitor blocking agents, a non-ionic detergent other than TWEEN 20, a hot start component, and/or a passive reference dye in a buffered solution. In some additional embodiments, such buffer solutions may comprise glycerol, DMSO, and/or a second salt.

In an embodiment, the kit can further comprise, in addition to the composition or master mix, at least one primer pair specific for PCR amplification of a nucleic acid target, and/or at least one probe specific for the amplified nucleic acid target. For example, the probe can be a TaqMan^{™} probe, a HydrolEasy^{™} probe, a minor groove binding (MGB) probe, a locked nucleic acid (LNA) probe, a SYBR Green or SYBR GreenER^{™} probe, or a cycling probe technology (CPT) probe.

In another embodiment, the kit can further comprise a control nucleic acid sample, and at least one primer pair specific for PCR amplification of a nucleic acid target in the control nucleic acid sample, and/or at least one probe specific for the amplified nucleic acid target in the control nucleic acid sample. A probe for detecting the amplification can also be included in the kit.

Components of the kit other than the composition may be provided in individual containers or in a single container, as appropriate. In some embodiments, instructions, manuals, and/or protocols for using the kit advantageously can be provided. Many assays are suitable for use in synthesizing target nucleic acids using the disclosed compositions and reaction mixtures and are contemplated herein as would be understood by one of skill in the art.

Also disclosed herein are methods of using the described compositions and kits. The compositions, reaction mixtures, and kits as provided herein can be used in a variety of nucleic acid synthesis-based assays to detect, quantify, and/or characterize a nucleic acid target or targets. In some embodiments, such assays comprise mixing any of the compositions provided herein with a nucleic acid sample and one or more primers and performing said assay. In certain embodiments, the methods involve nucleic acid amplification, such as by a polymerase chain reaction (PCR).

In certain aspects, the methods provided herein can also employ a hydrolysis probe for the detection of nucleic acids. Hydrolysis probes take advantage of the 5' exonuclease activity of some polymerases. During the extension or elongation phase of a PCR reaction, a polymerase, such as Taq polymerase, uses an upstream primer as a binding site and then extends. The hydrolysis probe is then cleaved during polymerase extension at its 5' end by the 5'-exonuclease activity of the polymerase.

The terms "upstream" and "downstream" are used herein in relation to the synthesis of the nascent strand that is primed by a target-specific primer. Thus, for example, a target-specific probe hybridized to a region of the target nucleic acid that is "downstream" of the region of the target nucleic acid to which the primer is hybridized is located 3 ' of the primer and will be in the path of a polymerase extending the primer in a 5' to 3' direction.

The TaqMan^{®} assay (see, e.g. , U.S. Patent 5,210,015, incorporated herein by reference in its entirety) is an example of a hydrolysis-probe based assay. In the TaqMan^{®} assay, hydrolysis probes are typically labeled with a reporter on the 5' end and a quencher on the 3' end. When the reporter and quencher are fixed onto the same probe, they are forced to remain in close proximity. This proximity effectively quenches the reporter signal, even when the probe is hybridized to the target sequence. The hydrolysis probes are cleaved during polymerase extension at their 5' end by the 5'-exonuclease activity of Taq. When this occurs, the reporter fluorophore is released from the probe, and subsequently, is no longer in close proximity to the quencher. This produces a perpetual increase in reporter signal with each extension phase as the PCR reaction continues cycling. In order to achieve maximal signal with each cycle, hydrolysis probes are often designed with a Tm that is roughly 10°C higher than the primers in the reaction. Uses of the real-time hydrolysis probe reaction are also described in U.S. Patent Nos. 5,538,848, 6,653,473, 7,485,442 and 7,205,105, the disclosures of all of which are incorporated herein by reference in their entireties. In some embodiments, the methods disclosed herein involve the use of a TaqMan assay for nucleic acid analysis and/or detection. For example, the compositions of the present disclosure can be used in methods involving TaqMan probes and/or assays. Such assays can include, but are not limited to gene expression assays (e.g., TaqMan^{™} Gene Expression Assays), copy number variation assays (e.g., TaqMan^{™} Copy Number Assays), genotyping assays (e.g., TaqMan^{™} Drug Metabolism Genotyping Assays or TaqMan^{™} SNP Genotyping Assays), miRNA assays (e.g., TaqMan^{™} MicroRNA Assays) or RNA quantitation assays (e.g., two-step reverse transcription-polymerase chain reaction assays), and TaqMan^{™} Low Density Array Assays.

As used herein, the term "reaction vessel" generally refers to any container in which a reaction can occur in accordance with the present teachings. In some embodiments, a reaction vessel may be a microtube, for example, but not limited to, a 0.2 mL or a 0.5 mL reaction tube such as a MicroAmp^{™} Optical tube (Applied Biosystems^{™}, Thermo Fisher Scientific) or a micro-centrifuge tube, or other containers of the sort in common practice in molecular biology laboratories. In some embodiments, a reaction vessel may be a well in a microtiter plate (e.g., 96-well plate, 384-well plate) such as a TaqMan^{™} Array plate (Applied Biosystems^{™}; Thermo Fisher Scientific), a spot on a glass slide, a well in an Applied Biosystems^{™} TaqMan^{™} Array Card or Plate (Thermo Fisher Scientific) or a through-hole of an Applied Biosystems^{™} TaqMan^{™} OpenArray^{™} plate (Thermo Fisher Scientific). For example, a plurality of reaction vessels may reside on the same support. In some embodiments, lab-on-a-chip-like devices, available for example from Caliper and Fluidigm, can provide for reaction vessels. In some embodiments, various microfluidic approaches may be employed. It will be recognized that a variety of reaction vessels are available in the art and fall within the scope of the present teachings.

The terms "amplicon" and "amplification product" or "amplified product" as used herein generally refer to the product of an amplification reaction. An amplicon may be double-stranded or single-stranded, and may include the separated component strands obtained by denaturing a double-stranded amplification product. In certain embodiments, the amplicon of one amplification cycle can serve as a template in a subsequent amplification cycle.

As used herein, the term "amplifying" refers to any means by which at least a part of a target polynucleotide, target polynucleotide surrogate, or combinations thereof, is reproduced, typically in a template-dependent manner, including without limitation, a broad range of techniques for amplifying nucleic acid sequences, either linearly or exponentially. Any of several methods can be used to amplify the target polynucleotide. Any in vitro means for multiplying the copies of a target sequence of nucleic acid can be utilized. These include linear, logarithmic, or any other amplification method. Exemplary methods include polymerase chain reaction (PCR), partial destruction of primer molecules (see, e.g., PCT Application Pub WO 2006/087574), ligase chain reaction (see, e.g., Wu, et al. Genomics 4:560-569 (1990) and Barany, et al. Proc. Natl. Acad. Sci. USA 88:189-193 (1991), Qβ RNA replicase systems (see, e.g., WO 1994/016108), RNA transcription-based systems (e.g., TAS, 3SR), rolling circle amplification (RCA) (see, e.g., U.S. Pat. No. 5,854,033; Lizardi, et al. Nat. Genet. 19:225-232 (1998); and Banér, et al. Nucleic Acid Res. 26: 5073-5078 (1998)), and strand displacement amplification (SDA) (Little, et al. Clin. Chem. 45:777-784 (1999)), among others. Many systems are suitable for use in amplifying target nucleic acids and are contemplated herein as would be understood by one of skill in the art. In some embodiments of the present methods, nucleic acid amplification reaction can be performed by PCR. In some embodiments, the PCR can be quantitative PCR (qPCR). In certain embodiments, the qPCR can be performed by real time PCR. In other embodiments, the PCR can be endpoint PCR. In other embodiments, the PCR can be digital PCR. In yet other embodiments, the PCR can comprise thermal cycling. In some embodiments, the thermal cycling can be optimized for fast thermal cycling.

In some embodiments, the methods for amplifying a nucleic acid by PCR comprises adding a composition as provided herein comprising a thermostable DNA polymerase, a salt, an antifoam agent, and dNTPs and/or dNTP derivatives to a reaction vessel; adding a nucleic acid sample and a primer to the reaction vessel to form a reaction mixture; and performing PCR on the nucleic acid sample. In some embodiments, the PCR continues to occur for up to 72 hours (e.g., for up to 4 hours, 8 hours, 12 hours, 24 hours, 36 hours, 48 hours, 60 hours, or 72 hours) following the addition of the composition, nucleic acid sample, and primer to the reaction vessel.

In some embodiments, the methods for amplifying a target nucleic acid can be multiplex PCR amplifications in which multiple targets are simultaneously amplified. The number of targets amplified can be up to as many as 2 targets, 5 targets, 10 targets 25 targets, 50 targets, 100 targets, 1000 targets, 5000 targets, and so on, including all numbers in between. In some embodiments, one of the multiplexed targets is an endogenous or an exogenous internal positive control for amplification. In some embodiments, the methods for detecting a target nucleic acid can be multiplex PCR and/or detection assays in which multiple targets are simultaneously detected. In certain embodiments, the number of targets amplified and/or detected can be up to 25 targets, 10 targets, 8 targets, 6 targets, 5 targets, 4 targets, 3 targets, or 2 targets. In an embodiment, one of the multiplexed targets is an endogenous or an exogenous internal positive control for amplification and/or detection. In some embodiments, a multiplicity of targets are simultaneously amplified and detected. In some embodiments, a multiplicity of targets are amplified and detected in the same reaction vessel. In some embodiments, various TaqMan^{™} probe reporter dye and passive dye options can be combined for multiplex PCR. For example, TaqMan^{™} probes with FAM^{™}, VIC^{™}, and ABY^{™} reporter dyes in combination with a PCR composition containing ROX passive reference dye may be used for a 3-plex multiplex PCR amplification and detection assay. For another example, TaqMan^{™} probes with FAM^{™}, VIC^{™}, ABY^{™}, and JUN^{™} reporter dyes in combination with a PCR composition containing MUSTANG PURPLE^{™} passive reference dye may be used for a 4-plex multiplex PCR amplification and detection assay. In some embodiments, nucleic acid synthesis (such as a nucleic acid amplification reaction or a PCR) and nucleic acid detection (e.g., of an amplicon) can occur simultaneously. Accordingly, in some embodiments, nucleic acid synthesis and nucleic acid detection occur in the same reaction vessel.

In general, PCR thermal cycling includes an initial denaturing step at high temperature, followed by a repetitive series of temperature cycles designed to allow template denaturation, primer annealing, and extension of the annealed primers by the polymerase. Generally, the samples are heated initially for about 2 to 10 minutes at a temperature of about 95° C to denature the double stranded DNA sample. Then, in the beginning of each cycle, the samples are denatured for about 10 to 60 seconds, depending on the samples and the type of instrument used. After denaturing, the primers are allowed to anneal to the target DNA at a lower temperature, typically from about 40° C to about 60° C for about 20 to 60 seconds. Extension of the primers by the polymerase is often carried out at a temperature ranging from about 60° C to about 72° C. The amount of time used for extension will depend on the size of the amplicon and the type of enzymes used for amplification and is readily determined by routine experimentation. Additionally, the annealing step can be combined with the extension step, resulting in a two-step cycling. Thermal cycling may also include additional temperature shifts in PCR assays. The number of cycles used in the assay depends on many factors, including the primers used, the amount of sample DNA present, and the thermal cycling conditions. The number of cycles to be used in any assay may be readily determined by one skilled in the art using routine experimentation. Optionally, a final extension step may be added after the completion of thermal cycling to ensure synthesis of all amplification products.

In one embodiment, exemplary thermal cycling conditions for PCR amplifications using the compositions and reaction mixtures disclosed herein are as follows:
UNG Step (Optional): 50 °C, 2 min (e.g., to prevent amplicon/carry over contamination from previous PCRs)
Activation: 95 °C, 20 sec
Denaturation: 95 - 97 °C/1-3 sec
Extension: 60 - 62 °C/ 20 - 30 sec (x 40 cycles)

In one embodiment, when the composition disclosed here is used for the TaqMan^{®} Low Density Array (TLDA) platform, an activation of 92 °C for 10 minutes is recommended.

The composition and reaction mixtures provided may also be used for amplification reactions in which a limited number of cycles occur (for example, but not limited to 2, 3, 4, 5, 6, 7, 8, 9, 10, 11, 12, 13, 14, or 15 cycles of amplification), such as in pre-amplification reactions. In certain embodiments, a pre-amplification step is performed using the provided compositions and reaction mixtures (See, for e.g., US Patent No. 9, 206, 475, the disclosure of which is incorporated by reference in its entirety). In some embodiments, a pre-amplification is performed with a pair of universal forward and reverse primers. In some embodiments, pre-amplification is performed for under than 20 cycles, for example, 2-18 cycles. In some embodiments, the pre-amplification step is truncated prior to reaching an amplification reaction plateau. In some embodiments, the methods disclosed herein can include a pre-amplification step which is performed prior to amplification using the compositions and reaction mixtures provided herein.

PCR with the disclosed composition can be performed on "standard" PCR instrumentation , e.g., Applied Biosystems^{™} 7900HT, 7500, and 7300 standard PCR systems, or on "Fast" PCR instrumentation, e.g., Applied Biosystems^{™} StepOne, StepOne Plus, 7500 and 7900HT Fast Real-Time PCR systems. In some embodiments, PCR with the disclosed compositions comprises thermal cycling using an instrument such as, without limitation, a GeneAmp^{®} PCR System 9700, 9600, 2700 or 2400 thermocycler, an Applied Biosystems^{®} ViiA^{™} 7 Real-Time PCR System, a QuantStudio^{™} 12K Flex Real-Time PCR System, a QuantStudio^{™} Dx Real-Time PCR System, a QuantStudio^{™} 6 Flex Real-Time PCR System, a QuantStudio^{™} 7 Flex Real-Time PCR System, a QuantStudio^{™} 3 Real-Time PCR System, a QuantStudio^{™} 5 Real-Time PCR System, and the like (all from Thermo Fisher Scientific). Other examples of spectrophotometric thermal cyclers for use in the methods include, but are not limited to, Bio-Rad ICycler IQ^{™}, Cepheid SmartCycler^{™} II, Corbett Research Rotor-Gene 3000, Idaho Technologies R.A.P.I.D.^{™}, MJ Research Chromo 4^{™}, Roche Applied Science LightCycler^{™}, Roche Applied Science LightCycler^{®}2.0, Stratagene Mx3000P^{™}, and Stratagene Mx4000^{™}.

In some aspects, the compositions, kits, and methods provided herein offer an advantage over standard or traditional compositions, kits and methods (such as other commercially available master mixes or kits) used for nucleic acid synthesis and/or detection. In some embodiments, such advantages include, but are not limited, to any of the following:
a) can be used to amplify nucleic acids (e.g., by real time qPCR) directly from unprocessed samples (e.g., can amplify targets directly from saliva and whole blood)
b) improves the accuracy of copy number variation results;
c) provides increased tolerance to various PCR inhibitors;
e) provides increased specificity and sensitivity;
f) can be used with fast thermal cycling protocols for quicker read-outs; and/or
g) allows for the capability to multiplex (e.g., (i) can amplify a multiplicity of targets using a single sample; two or more targets in one sample or (ii) can amplify multiple samples; two targets in two different samples) in a single reaction at substantially the same time.

In some embodiments, the accuracy of copy number variation in PCRs is improved by at least 1%, at least 5%, at least 10%, at least 15%, at least 20% or at least 25% when using the compositions, kits, and methods provided herein compared to copy number variation observed in PCRs using standard or traditional compositions, kits, and method. In some embodiments, the input detection sensitivity in PCRs is increased by at least 10 fold, 50 fold, 100 fold, 500 fold, or 1000 fold when using the compositions, kits, and methods provided herein compared to the input detection sensitivity observed in PCRs using standard or traditional compositions, kits, and method. In some embodiments, the specificity and sensitivity in PCRs is at least 80%, 85%, 90%, 95% or 99% accurate for genotyping when using the compositions, kits, and methods provided herein. In some embodiments, any of the advantages (a) through (g), listed above, are at least comparable to those observed using standard or traditional compositions, kits and methods for nucleic acid synthesis and/or detection.

As used herein, "nucleotide" refers to a base-sugar-phosphate combination. A "nucleoside" refers to a base-sugar combination. Nucleotides are monomeric units of a nucleic acid sequence (e.g., DNA and RNA). The term nucleotide includes mono-, di- and triphosphate forms of deoxyribonucleosides and ribonucleosides and their derivatives. dNTPs may be unlabeled, or they may be detectably labeled by coupling them by methods known in the art with radioisotopes (e.g., H³, C¹⁴, P³² or S³⁵), vitamins (e.g., biotin), fluorescent moieties (e.g., fluorescein, rhodamine, Texas Red, or phycoerythrin), chemiluminescent labels, dioxigenin and the like. Labeled dNTPs may be obtained commercially, for example from Thermo Fisher Scientific or Sigma-Aldrich Company.

As used herein, "polynucleotide" and "oligonucleotide" refer to a synthetic or biologically produced molecule comprising a covalently linked sequence of nucleotides which may be joined by a phosphodiester bond between the 3' position of the pentose of one nucleotide and the 5' position of the pentose of the adjacent nucleotide. In addition, a polynucleotide or oligonucleotide may contain modified or non-naturally occurring sugar residues (e.g., arabinose) and/or modified base residues. A polynucleotide or oligonucleotide may also comprise blocking groups that prevent the interaction of the molecule with particular proteins, enzymes or substrates.

As used herein, "nucleic acid" includes compounds having a plurality of natural nucleotides and/or non-natural (or "derivative") nucleotide units. A "nucleic acid" can further comprise non-nucleotide units, for example peptides. "Nucleic acid" therefore encompasses compounds such as DNA, RNA, peptide nucleic acids, phosphothioate-containing nucleic acids, phosphonate-containing nucleic acids and the like. There is no particular limit as to the number of units in a nucleic acid, provided that the nucleic acid contains 2 more nucleotides, nucleotide derivatives, or combinations thereof, specifically 5, 10, 15, 25, 50, 100, or more. Nucleic acids can encompass both single and double-stranded forms, and fully or partially duplex hybrids (e.g., RNA-DNA, RNA-PNA, or DNA-PNA).

As used herein, the term "primer" may refer to more than one primer and refers to an oligonucleotide, whether occurring naturally, as in a purified restriction digest, or produced synthetically, which is capable of acting as a point of initiation of synthesis along a complementary strand when placed under conditions in which synthesis of a primer extension product which is complementary to a nucleic acid strand is catalyzed. Such conditions include the presence of four different deoxyribonucleoside triphosphates and a polymerization-inducing agent such as DNA polymerase or reverse transcriptase, in a suitable buffer ("buffer" includes substituents which are cofactors, or which affect pH, ionic strength, etc.), and at a suitable temperature. The primer is preferably single-stranded for maximum efficiency in amplification. A primer is typically 11 bases or longer; more specifically, a primer is 17 bases or longer, although shorter or longer primers may be used depending on the need. As will be appreciated by those skilled in the art, the oligonucleotides may be used as one or more primers in various extension, synthesis or amplification reactions.

The complement of a nucleic acid sequence as used herein refers to a oligonucleotide or a polynucleotide which, when aligned with the nucleic acid sequence such that the 5' end of one sequence is paired with the 3' end of the other, is in "antiparallel association." Certain bases not commonly found in natural nucleic acids may be included in the nucleic acids of the present invention and include, for example, inosine and 7-deazaguanine. Complementarity need not be perfect; stable duplexes may contain mismatched base pairs or unmatched bases. Those skilled in the art of nucleic acid technology can determine duplex stability empirically considering a number of variables including, for example, the length of the oligonucleotide, base composition and sequence of the oligonucleotide, ionic strength, and incidence of mismatched base pairs.

Stability of a nucleic acid duplex is measured by the melting temperature ("Tm"). The Tm of a particular nucleic acid duplex under specified conditions is the temperature at which half of the base pairs have disassociated.

When referring to a thermostable DNA polymerase, one unit of activity is the amount of enzyme that will incorporate 10 nanomoles of dNTPs into acid-insoluble material (i.e., DNA or RNA) in 30 minutes under standard primed DNA synthesis conditions.

As used herein, the term "target," "target sequence," "target template," or "target nucleic acid sequence" refers to a nucleic acid sequence or region of a nucleic acid which is to be either amplified, detected, or both. Typically, in a PCR, the target sequence resides between the two primer sequences used for amplification.

The term "or combinations thereof" as used herein refers to all permutations and combinations of the listed terms preceding the term. For example, "A, B, C, or combinations thereof" is intended to include at least one of: A, B, C, AB, AC, BC, or ABC, and if order is important in a particular context, also BA, CA, CB, ACB, CBA, BCA, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

Exemplary samples of nucleic acids include DNA and/or RNA. In one embodiment, the nucleic acid is an isolated and/or purified nucleic acid. An isolated or purified nucleic acid is substantially free of other components such as proteins, polysaccharides, or other cellular, bacterial, or viral components. In another embodiment, the nucleic acid is not isolated or purified. In some embodiments, the nucleic acid sample is within a biological sample. For example, the nucleic acid may be present in a complex mixture, such as a crude lysate or whole cell extract. In another embodiment, the nucleic acid may be in situ and exist within its normal cellular, bacterial, or viral environment.

The target nucleic acid may be obtained from any source, and may comprise any number of different compositional components. For example, the target may be a nucleic acid (e.g., DNA or RNA), messenger RNA (mRNA), transfer RNA (tRNA), small interfering RNA (siRNA), microRNA (miRNA), or other mature small RNA, and may comprise nucleic acid analogs or other nucleic acid mimics. The target may be methylated, non-methylated, or both. The target may be bisulfite-treated and non-methylated cytosines converted to uracil. Further, it will be appreciated that "target nucleic acid" may refer to the target nucleic acid itself, as well as surrogates thereof, for example, amplification products and native sequences.

The nucleic acid samples of the present teachings may be obtained from any number of biological sources, including without limitation, viruses, archae, protists, prokaryotes and eukaryotes, for example, from a biological sample obtained from a eukaryotic organism, most preferably a mammal such as a primate e.g., chimpanzee or human; cow; dog; cat; a rodent, e.g., guinea pig, rat, mouse; rabbit; or a bird; reptile; or fish. The nucleic acid may be obtained from cells, tissues, organs, or organisms in different developmental stages. The nucleic acid samples may also be obtained from cancer cells and precancerous cells obtained from animals, including humans. The nucleic acid may also be obtained from cell culture lines, including transformed and non-transformed cell culture lines. Nucleic acid samples can be extracted from a variety of sources. These include, but are not limited to, for example clothing, soil, paper, metal surfaces, air, water, plant parts, as well as human and/or animal skin, hair, blood, serum, feces, milk, saliva, urine, and/or other secretory fluids.

It will be appreciated that nucleic acid samples and target nucleic acids may be extracted from biological and other samples using any of a variety of procedures known in the art, for example, DNA Extract All Reagents Kit (Applied Biosystems^{™}, Thermo Fisher Scientific), MagMax^{™} Sample Preparation Systems (Thermo Fisher Scientific), TargetPrep RNA FFPE Sample Preparation Kit (Abbott Laboratories), RecoverAll^{™} Total Nucleic Acid Isolation Kit for FFPE and PureLink^{™} FFPE RNA Isolation Kit (Ambion^{™}, Thermo Fisher Scientific), the ABI Prism^{®} 6100 Nucleic Acid PrepStation and the ABI Prism^{®} 6700 Automated Nucleic Acid Workstation (Applied Biosystems, Thermo Fisher Scientific), and the like.

"Biological sample" includes sections of tissues such as biopsy and autopsy samples, and frozen sections taken for histologic purposes. Such samples include blood and blood fractions or products (e.g., serum, platelets, red blood cells, and the like), lymph, bone marrow, sputum, bronchoalveolar lavage, amniotic fluid, hair, skin, cultured cells, e.g., primary cultures, explants, and transformed cells, stool, urine, etc. Prior to target nucleic acid preparation, biological samples may be fresh, frozen or formalin- or paraformalin-fixed paraffin-embedded tissue (FFPE).

The PCR compositions provided are compatible with crude nucleic acid extraction methods and can tolerate PCR inhibitors that are carried over from sample preparation. The PCR compositions are compatible with crude cell or tissue lysates such as those prepared from, for example, whole blood in liquid form, whole blood dried on paper, buccal swabs (e.g., oral mucosa), raw saliva, urine, serum, hair follicles, plant leaf, and FFPE.

As used herein, the term "crude sample" refers to a specimen of biological origin suspected of containing nucleic acids, which has not undergone procedures for the isolation or purification of those nucleic acids. For example, a sample of blood or urine is a crude sample. Further, a sample of blood spotted on paper containing a lysis reagent, such as FTA paper (commercially available from Whatman), is also considered a crude sample. A buccal swab of cheek cells is another example of a crude sample. Crude samples include, but are not limited to, blood, diluted blood, blood on paper, buccal swabs, and buccal swabs on a substrate for samples storage, such as FTA paper. The cells in a crude sample can optionally be lysed to create a "crude lysate." One of skill in the art will recognize an enormous variety of other crude samples or crude lysates whose analysis would be facilitated by the present teachings.

Following amplification or synthesis, the amplified or synthesized nucleic acid fragments may be isolated for further use or characterization. This step is usually accomplished by separation of the amplified or synthesized nucleic acid fragments by size or by any physical or biochemical means including gel electrophoresis, capillary electrophoresis, chromatography (including sizing, affinity and immunochromatography), density gradient centrifugation and immunoadsorption. An exemplary method is separation of nucleic acid fragments by gel electrophoresis, which provides a rapid and highly reproducible means of sensitive separation of a multitude of nucleic acid fragments, and permits direct, simultaneous comparison of the fragments in several samples of nucleic acids.

In one embodiment, one or more of the amplified or synthesized nucleic acid fragments are removed from the gel which was used for identification (see above), according to standard techniques such as chemical extraction, electroelution, or physical excision. The isolated unique nucleic acid fragments may then be inserted into standard vectors, including expression vectors, suitable for transfection or transformation of a variety of prokaryotic (bacterial) or eukaryotic (yeast, plant or animal including human and other mammalian) cells. Alternatively, nucleic acids produced by the methods may be further characterized, for example by sequencing (i.e., determining the nucleotide sequence of the nucleic acid fragments), by methods described below and others that are standard in the art (see, e.g., U.S. Pat. Nos. 4,962,022 and 5,498,523, which are directed to methods of DNA sequencing). Classical sequencing methods may also be employed such as the Sanger chain termination method (Sanger, F., et al. Proc. Natl. Acad. Sci. USA 74: 5463-5467 (1977)) and the Maxam and Gilbert chemical cleavage method (Maxam, A. M. and Gilbert, W. Proc. Natl. Acad. Sci. USA 74: 560-564 (1977)).

In one embodiment, nucleic acids produced by the methods may be further characterized by next generation sequencing. As used herein, the term "next generation sequencing" or "NGS" generally refers to high throughput sequencing technologies, including, but not limited to, massively parallel signature sequencing, high throughput sequencing, sequencing by ligation (e.g., SOLiD sequencing), proton ion semiconductor sequencing, DNA nanoball sequencing, single molecule sequencing, and nanopore sequencing.

Suitable DNA polymerases for amplification and/or sequencing can include any of those previously disclosed herein. In some embodiments, such polymerase include, but are not limited to, *Thermus thermophilus* (Tth) DNA polymerase, *Thermus aquaticus* (Taq) DNA polymerase, *Thermotoga neopolitana* (Tne) DNA polymerase, *Thermotoga maritima* (Tma) DNA polymerase, *Thermococcus litoralis* (Tli or VENT^{™}) DNA polymerase, *Pyrococcus furiosus* (Pfu) DNA polymerase, DEEPVENT^{™} DNA polymerase, *Pyrococcus woosii* (Pwo) DNA polymerase, *Pyrococcus* sp KOD2 (KOD) DNA polymerase, *Bacillus sterothermophilus* (Bst) DNA polymerase, *Bacillus caldophilus* (Bca) DNA polymerase, *Sulfolobus acidocaldarius* (Sac) DNA polymerase, *Thermoplasma acidophilum* (Tac) DNA polymerase, *Thermus flavus* (Tfl/Tub) DNA polymerase, *Thermus ruber* (Tru) DNA polymerase, *Thermus brockianus* (DYNAZYME^{™}) DNA polymerase, *Methanobacterium thermoautotrophicum* (Mth) DNA polymerase, *Mycobacterium* DNA polymerase (Mtb, Mlep), *E. coli* pol I DNA polymerase, Klenow fragment, T5 DNA polymerase, T7 DNA polymerase, and generally pol I type DNA polymerases; mutants, variants and derivatives thereof, and combinations of the foregoing.

Suitable nucleic acid polymerases may be mesophilic or thermophilic, and are preferably thermophilic and thermostable. As used herein, the term "thermostable nucleic acid polymerase" refers to an enzyme which is relatively stable to heat when compared, for example, to nucleotide polymerases from *E. coli* and which catalyzes the polymerization of nucleoside triphosphates. Generally, the enzyme will initiate synthesis at the 3'-end of the primer annealed to the target sequence, and will proceed in the 5'-direction along the template, and if possessing a 5' to 3' nuclease activity, hydrolyzing intervening, annealed probe to release both labeled and unlabeled probe fragments, until synthesis terminates. A representative thermostable enzyme isolated from *Thermus aquaticus* (Taq) is described in U.S. Pat. No. 4, 889,818 and a method for using it in conventional PCR is described in Saiki et al., 1988, Science 239:487.

Suitable mesophilic DNA polymerases include Pol I family of DNA polymerases (and their respective Klenow fragments) any of which may be isolated from organism such as *E. coli, H. influenzae, D. radiodurans, H. pylori, C. aurantiacus, R. prowazekii, T pallidum, Synechocystis* sp., *B. subtilis, L. lactis, S. pneumoniae, M. tuberculosis, M. leprae, M. smegmatis,* Bacteriophage L5, phi-C31, T7, T3, T5, SP01, SP02, mitochondrial from S. *cerevisiae* MIP-1, and eukaryotic C. *elegans,* and *D. melanogaster* (Astatke, M. et al., 1998, J Mol. Biol. 278, 147-165), pol III type DNA polymerase isolated from any sources, and mutants, derivatives or variants thereof, and the like.

In certain embodiments, the nucleic acid polymerase has 5'→3' exonuclease activity. As defined herein, "5'-3' nuclease activity" or "5' to 3' nuclease activity" refers to that activity of a template-specific nucleic acid polymerase including either a 5'-3' exonuclease activity traditionally associated with some DNA polymerases whereby nucleotides are removed from the 5' end of an oligonucleotide in a sequential manner, (i.e., *E. coli* DNA polymerase I has this activity whereas the Klenow fragment does not), or a 5'-3' endonuclease activity wherein cleavage occurs more than one phosphodiester bond (nucleotide) from the 5' end, or both. Taq DNA polymerase has a DNA synthesis-dependent, strand replacement 5'-3' exonuclease activity (see Gelfand, "Taq DNA Polymerase" in PCR Technology: Principles and Applications for DNA Amplification, Erlich, Ed. , Stockton Press, N.Y.

(1989), Chapter 2). In some embodiments methods comprising reverse transcription polymerase chain reaction (RT-PCR) are contemplated herein. In such methods, the compositions and reaction mixtures provided herein may further comprise an enzyme that has reverse transcriptase activity. Suitable enzymes having reverse transcriptase activity can be, for example, retroviral reverse transcriptases such as Moloney Murine Leukemia Virus (M-MLV) reverse transcriptase, Rous Sarcoma Virus (RSV) reverse transcriptase, Human Immunodeficiency Virus (HIV) reverse transcriptase, AMV reverse transcriptase, RAV reverse transcriptase, MAV reverse transcriptase, ASLV reverse transcriptases, as well as Lentivirus reverse transcriptases, or corresponding mutants, variants or derivatives thereof having reverse transcriptase activity. As used herein, "mutants, variants, or derivatives" refer to all permutations of a chemical species, which may exist or be produced, that still retains the definitive chemical activity of that chemical species. Some preferred enzymes for use in the invention include those that are RNase H+ enzymes such as, for example, RNase H+ M-MLV or RNase H+ AMV reverse transcriptases. Alternatively, the reverse transcriptases may have reduced, substantially reduced, or eliminated RNase H activity (see, e.g., U.S. Pat. No. 7,078,208, the disclosure of which is fully incorporated by reference in its entirety). RNase H is a processive 5' and 3' ribonuclease that is specific for the RNA strand of RNA-DNA hybrids (Perbal, A Practical Guide to Molecular Cloning, New York: Wiley & Sons (1984)). RNase H activity may be determined by a variety of assays, such as those described, for example, in U.S. Pat. No. 5,244,797, in Kotewicz, M. L., et al., Nucl. Acids Res. 16:265 (1988) and in Gerard, G. F., et al., FOCUS 14(5):91 (1992).

The reverse transcriptase may comprise a mutation as compared to the naturally-occurring reverse transcriptase. For example, the reverse transcriptase may be modified to contain a mutation that provides increased reverse transcriptase stability and/or functionality. Suitable enzymes may also include those in which terminal deoxynucleotidyl transferase (TdT) activity has been reduced, substantially reduced, or eliminated. Reverse transcriptases which exhibit such increased or decreased functionalities are described in, for example, U.S. Pat. Nos. 7,056,716 and 7,078,208.

As presented herein, assembled PCR compositions provided herein are effective at reducing PCR run times as compared to an equivalent PCR with a commercially available master mix. This was particularly the case when the nucleic acid sample added to the PCR was a crude lysate and/or the target sequence was present at a low copy number. In some embodiments, this reduction of PCR run times is demonstrated by lower Ct values. In some embodiments, the Ct value is at least 10 fold, at least 50 fold, at least 100 fold, at least 500 fold, or at least 1000 fold lower when using assembled PCR compositions provided herein compared to an equivalent PCR with a commercially available composition or master mix.

As used herein the term "Ct" or "Ct value" refers to threshold cycle and signifies the cycle of a PCR amplification assay in which signal from a reporter that is indicative of amplicon generation (e.g., fluorescence) first becomes detectable above a background level. In some embodiments, the threshold cycle or "Ct" is the cycle number at which PCR amplification becomes exponential. In one embodiment, the signal from a reporter, such as fluorescence, is described as delta Rn. As used herein, the term "dRn" or "delta Rn" refers to the difference in the normalized reporter signal (Rn) subtracted from the background signal (baseline) which is then normalized by a passive reference signal. Delta Rn can be determined by the formula Rn⁺ - Rn⁻, where Rn⁺ is the Rn value for a reaction involving all components, including the template, and Rn⁻ is the value for an unreacted sample.

According to various embodiments, a Ct value may be determined using a derivative of a PCR curve. For example, a first, second, or nth order derivative method may be performed on a PCR curve in order to determine a Ct value. In various embodiments, a characteristic of a derivative may be used in the determination of a Ct value. Such characteristics may include, but are not limited by, a positive inflection of a second derivative, a negative inflection of a second derivative, a zero crossing of the second derivative, or a positive inflection of a first derivative. In various embodiments, a Ct value may be determined using a thresholding and baselining method. For example, an upper bound to an exponential phase of a PCR curve may be established using a derivative method, while a baseline for a PCR curve may be determined to establish a lower bound to an exponential phase of a PCR curve. From the upper and lower bound of a PCR curve, a threshold value may be established from which a Ct value is determined. Other methods for the determination of a Ct value known in the art, for example, but not limited by, various embodiments of a fit point method, and various embodiments of a sigmoidal method (See, e.g., U.S. Patent Nos. 6,303,305; 6,503,720; 6,783,934, 7,228,237 and U.S. Application No. 2004/0096819; the disclosures of which are herein incorporated by reference in their entireties).

As used herein the terms "annealing" and "hybridization" are used interchangeably and mean the complementary base-pairing interaction of one nucleic acid with another nucleic acid that results in formation of a duplex, triplex, or other higher-ordered structure. In some embodiments, the primary interaction is base specific, e.g., A/T and G/C, by Watson/Crick and Hoogsteen-type hydrogen bonding. In some embodiments, base-stacking and hydrophobic interactions may also contribute to duplex stability. Conditions for hybridizing nucleic acid probes and primers to complementary and substantially complementary target sequences are well known, e.g., as described in Nucleic Acid Hybridization, A Practical Approach, B. Hames and S. Higgins, eds., IRL Press, Washington, D.C. (1985) and J. Wetmur and N. Davidson, Mol. Biol. 31:349 et seq. (1968). In general, whether such annealing takes place is influenced by, among other things, the length of the probes and the complementary target sequences, the pH, the temperature, the presence of mono- and divalent cations, the proportion of G and C nucleotides in the hybridizing region, the viscosity of the medium, and the presence of denaturants. Such variables influence the time required for hybridization. Thus, the preferred annealing conditions will depend upon the particular application. Such conditions, however, can be routinely determined by the person of ordinary skill in the art without undue experimentation. Further, in general probes and primers of the present teachings are designed to be complementary to a target sequence, such that hybridization of the target and the probes or primers occurs. It will be appreciated, however, that this complementarity need not be perfect; there can be any number of base pair mismatches that will interfere with hybridization between the target sequence and the single stranded nucleic acids of the present teachings. However, if the number of base pair mismatches is so great that no hybridization can occur under even the least stringent of hybridization conditions, the sequence is not a complementary target sequence. Thus, by "substantially complementary" herein is meant that the probes or primers are sufficiently complementary to the target sequence to hybridize under the selected reaction conditions.

The term "label" as used herein refers to any atom or molecule which can be used to provide a detectable signal, and which can be attached to a nucleic acid or protein. Labels may provide signals detectable by fluorescence, radioactivity, colorimetry, gravimetry, X-ray diffraction or absorption, magnetism, enzymatic activity, and the like. In some embodiments, the detectable signal is a quantifiable signal.

For example, a label can be any moiety that: (i) provides a detectable signal; (ii) interacts with a second label to modify the detectable signal provided by the first or second label; or (iii) confers a capture function, e.g. hydrophobic affinity, antibody/antigen, ionic complexation. The skilled artisan will appreciate that many different species of reporter labels can be used in the present teachings, either individually or in combination with one or more different labels. Exemplary labels include, but are not limited to, fluorophores, radioisotopes, Quantum Dots, chromogens, enzymes, antigens including but not limited to epitope tags, heavy metals, dyes, phosphorescence groups, chemiluminescent groups, electrochemical detection moieties, affinity tags, binding proteins, phosphors, rare earth chelates, and near-infrared dyes.

Suitable detectable labels include, for example, fluoresceins (e.g., 5-carboxy-2,7-dichlorofluorescein; 5-Carboxyfluorescein (5-FAM); 5-HAT (Hydroxy Tryptamine); 6-HAT; 6-JOE; 6-carboxyfluorescein (6-FAM); FITC); Alexa fluors (e.g., 350, 405, 430, 488, 500, 514, 532, 546, 555, 568, 594, 610, 633, 635, 647, 660, 680, 700, 750); BODIPY^{™} fluorophores (e.g., 492/515, 493/503, 500/510, 505/515, 530/550, 542/563, 558/568, 564/570, 576/589, 581/591, 630/650-X, 650/665-X, 665/676, FL, FL ATP, FI-Ceramide, R6G SE, TMR, TMR-X conjugate, TMR-X, SE, TR, TR ATP, TR-X SE), coumarins (e.g., 7-amino-4-methylcoumarin, AMC, AMCA, AMCA-S, AMCA-X, ABQ, CPM methylcoumarin, coumarin phalloidin, hydroxycoumarin, CMFDA, methoxycoumarin), calcein, calcein AM, calcein blue, calcium dyes (e.g., calcium crimson, calcium green, calcium orange, calcofluor white), Cascade Blue, Cascade Yellow; Cy^{™} dyes (e.g., 3, 3.18, 3.5, 5, 5.18, 5.5, 7), cyan GFP, cyclic AMP Fluorosensor (FiCRhR), fluorescent proteins (e.g., green fluorescent protein (e.g., GFP, EGFP), blue fluorescent protein (e.g., BFP, EBFP, EBFP2, Azurite, mKalama1), cyan fluorescent protein (e.g., ECFP, Cerulean, CyPet), yellow fluorescent protein (e.g., YFP, Citrine, Venus, YPet), FRET donor/acceptor pairs (e.g., fluorescein/tetramethylrhodamine, IAEDANS/fluorescein, EDANS/dabcyl, fluorescein/fluorescein, BODIPY^{™} FL/BODIPY^{™} FL, Fluorescein/QSY7 and QSY9), LysoTracker^{™} and LysoSensor^{™} (e.g., LysoTracker^{™} Blue DND-22, LysoTracker^{™} Blue-White DPX, LysoTracker^{™} Yellow HCK-123, LysoTracker^{™} Green DND-26, LysoTracker^{™} Red DND-99, LysoSensor^{™} Blue DND-167, LysoSensor^{™} Green DND-189, LysoSensor^{™} Green DND-153, LysoSensor^{™} Yellow/Blue DND-160, LysoSensor Yellow/Blue 10,000 MW dextran), Oregon Green (e.g., 488, 488-X, 500, 514); rhodamines (e.g., 110, 123, B, B 200, BB, BG, B extra, 5-carboxytetramethylrhodamine (5-TAMRA), 5 GLD, 6-Carboxyrhodamine 6G, Lissamine, Lissamine Rhodamine B, Phallicidine, Phalloidine, Red, Rhod-2, 5-ROX (carboxy-X-rhodamine), Sulphorhodamine B can C, Sulphorhodamine G Extra, Tetramethylrhodamine (TRITC), WT), Texas Red, Texas Red-X, VIC and other labels described in, e.g., US Pub. No. 2009/0197254), among others as would be known to those of skill in the art. Other detectable labels can also be used (see, e.g., U.S. Pat. Application Pub. No. 2009/0197254), as would be known to those of skill in the art.

Fluorescent reporter molecule--quencher molecule pairs have been incorporated onto oligonucleotide probes in order to monitor biological events based on the fluorescent reporter molecule and quencher molecule being separated or brought within a minimum quenching distance of each other. For example, probes have been developed where the intensity of the reporter molecule fluorescence increases due to the separation of the reporter molecule from the quencher molecule. Probes have also been developed which lose their fluorescence because the quencher molecule is brought into proximity with the reporter molecule. These reporter-quencher molecule pair probes have been used to monitor hybridization assays and nucleic acid amplification reactions, especially polymerase chain reactions (PCR), by monitoring either the appearance or disappearance of the fluorescence signal generated by the reporter molecule. (For example, see U.S. Pat. No. 6,030,787).

Exemplary reporter-quencher pairs may be selected from xanthene dyes, including fluoresceins, and rhodamine dyes. Many suitable forms of these compounds are widely available commercially with substituents on their phenyl moieties which can be used as the site for bonding or as the bonding functionality for attachment to an oligonucleotide. Another group of fluorescent compounds are the naphthylamines, having an amino group in the alpha or beta position. Included among such naphthylamino compounds are 1-dimethylaminonaphthyl-5-sulfonate, 1-anilino-8-naphthalene sulfonate and 2-p-touidinyl-6-naphthalene sulfonate. Other dyes include 3-phenyl-7-isocyanatocoumarin, acridines, such as 9-isothiocyanatoacridine and acridine orange; N-(p-(2-benzoxazolyl)phenyl) maleimide; benzoxadiazoles, stilbenes, pyrenes, and the like.

Preferably, reporter and quencher molecules are selected from fluorescein and rhodamine dyes. These dyes and appropriate linking methodologies for attachment to oligonucleotides are described in many references, e.g., Marshall, Histochemical J. , 7: 299-303 (1975); Menchen et al., U.S. Pat. No. 5,188,934; Menchen et al., European Patent Application 87310256.0; and Bergot et al., International Application PCT/US90/05565.

The section headings used herein are for organizational purposes only and are not to be construed as limiting the desired subject matter in any way. All literature cited in the disclosure, including but not limited to, patent, patent applications, articles, books and treatises are expressly incorporated by reference in their entirety for any purpose. In the event that any of the incorporated literature contradicts any term defined in this disclosure, this disclosure controls.

While the present teachings have been described in terms of these exemplary embodiments, the skilled artisan will readily understand that numerous variations and modifications of these exemplary embodiments are possible without undue experimentation. All such variations and modifications are within the scope of the current teachings. Aspects of the present teachings may be further understood in light of the following examples, which should not be construed as limiting the scope of the teachings in any way.

### EXAMPLES

### Example 1: Exemplary composition

An exemplary composition was formulated as a twofold concentrated stock solution comprising a thermostable DNA polymerase, a potassium salt, a magnesium salt, a silicon-based antifoam agent, dNTPS including a combination of dGTP and 7-deaza-dGTP, BSA, fish gelatin, a buffer solution, a hot start component, glycerol, a nonionic detergent other than TWEEN 20, and a passive reference dye. The exemplary PCR composition was tested in a number of assays as described in the Examples below.

### Example 2: Superior sensitivity of PCR of unpurified samples with the exemplary composition

The performance of the PCR composition of Example 1 in a TaqMan^{™} Copy Number Assay was compared with the performance of a commercially available composition, TaqMan^{™} Fast Advanced Master Mix ("MM"; Applied Biosystems^{™}, catalog no. 4444557) using two different nucleic acid samples. Assays were performed according to the manufacturer's protocols for the assay and MM, but substituting the exemplary composition for MM in one set of assays.

A crude nucleic acid sample was prepared from oral mucosa harvested using a buccal swab and a DNA Extract All Reagents Kit according to manufacturer's protocol (Applied Biosystems^{™}, catalog no. 4402599). Briefly, an oral mucosa sample was obtained by rubbing a 4N6FLOQSwab on the cheek of each side a participant's mouth. The swab was added to 200 microliters of lysis reagent and was incubated at 95°C for 3 minutes. After the sample cooled to room temperature, 200 microliters of stabilization reagent was added to each lysis solution sample to form a crude lysate of oral mucosa.

A TaqMan^{™} RNase P Copy Number Assay was performed using 2 microliters of the crude lysate and an additional amount of lysis solution. The reactions were performed in duplicate.

A single assembled 20 µL PCR contains the following components:
1 µL of RNase P assay (20X; forward and reverse PCR primer and TaqMan^{™} probe)
2 µL of crude lysate
1, 2, 3, 4, or 7 µL of lysis solution
10 µL TaqMan^{™} Fast Advanced Master Mix or the composition of Example 1
RNase-free water to total PCR volume of 20 µL

PCR was performed using an Applied Biosystems^{®} ViiA^{™} 7 Real-Time PCR System with the following thermal cycling conditions:
UNG Step (Optional): 50 °C, 2 minutes
Activation: 95 °C, 20 seconds
(Denaturation: 95°C/1 second;
Extension: 60°C/ 20 seconds ) x 40 cycles

Threshold cycle numbers (Ct) determined for each assay using the composition of Example 1 or the commercial MM are shown in FIG. 1. The composition of Example 1 resulted in amplification and detection of the single copy gene RNase P from 2 microliters of crude lysate from a buccal swab whereas no signal was obtained using the commercially available PCR master mix. Using the composition of Example 1, the RNase P signal was detected even in the presence of an additional 1, 2, 3, or 4 µL of lysis solution. This signal detection from a crude lysate plus additional lysis solution indicates that the reactions conditions would permit use of additional sample lysate in the assay. Accordingly, the exemplary composition provided herein supports more robust amplification from a crude lysate sample and is more tolerant to PCR inhibitors carried over from the crude extract than the commercially available master mix.

A fresh, raw saliva sample was used as the DNA source for a comparison of the exemplary composition with a commercially available PCR master mix in a copy number assay. The saliva sample was not pre-treated with a lysis solution or process. A TaqMan^{™} RNase P Copy Number Assay was performed using 1-9 microliters of the raw saliva. The reactions were performed in duplicate.

A single assembled 20 µL PCR contains the following components:
1 µL of RNase P assay (20X; forward and reverse PCR primer and TaqMan^{™} probe)
10 µL TaqMan^{™} Fast Advanced Master Mix or the composition of Example 1
1-9 µL of raw saliva
RNase-free water to total PCR volume of 20 µL

PCR was performed as ViiA^{™} 7 Real-Time PCR System with the thermal cycling conditions as indicated above. Threshold cycle numbers (Ct) determined for each assay using the composition of Example 1 or the commercial MM are shown in FIG. 2 for varying volumes of raw saliva. The composition of Example 1 resulted in amplification and detection of the single copy gene RNase P from all of the saliva samples tested whereas only the 1 µL saliva sample lead to a signal using the commercially available PCR master mix. Each of the 1, 2, 3, 4, and 9 µL samples of raw saliva generated a clear detectable response with the exemplary PCR composition (FIG. 2, upper panel, left box). The commercially available PCR master mix was not compatible with more than 5% of the reaction volume coming from the saliva. No signal was obtained from the 2, 3, 4, or 9 µL samples of raw saliva crude lysate with the commercially available PCR master mix (FIG. 2, upper panel, right box). Accordingly, the exemplary composition provided herein supports more robust amplification from a raw saliva sample and is more tolerant to PCR inhibitors carried over from the raw saliva than the commercially available master mix.

### Example 3: Genotyping of crude lysate sample with the exemplary composition

A crude nucleic acid sample was prepared from whole blood using a DNA Extract All Reagents Kit according to manufacturer's protocol. Briefly, samples of whole blood sample were incubated with the lysis solution at 95°C for 3 minutes, followed by addition of the stabilization solution to generate blood crude lysates.

A CYP2C19*.g.-806C>T TaqMan^{™} Genotyping Assay (Applied Biosystems TaqMan^{™} SNP Genotyping Assay ID No. C_469857_10) was performed using 2 microliters of each of the blood crude lysate and the exemplary PCR composition of Example 1. The genotyping assay was performed according to manufacturer's protocol. Fifty PCR cycles were performed for the assay.

The genotyping plot that resulted from analysis of the crude blood lysates is shown in FIG. 3. The VIC-labeled TaqMan^{™} MGB probe detected Allele 1 (the minor allele) and the FAM-labeled TaqMan^{™} probe detected Allele 2. In the graph of FIG. 3, the lines indicate the trace of signal intensity at each PCR cycle and the clusters show the intensities at the 40^{th} cycle. These results show that the exemplary composition provided herein can generate accurate genotyping results with crude nucleic acid samples. PCR and genotyping assay with the exemplary composition tolerates PCR inhibitors present in crude nucleic acid samples.

### Example 4: Comparison of crude lysate and purified DNA samples with the exemplary composition

A comparison between crude lysate and purified DNA samples on copy number variation was performed. Crude lysate was prepared from whole blood using Applied Biosystems^{™} DNA Extract All Reagents Kit according to manufacturer's protocol. Purified DNA sample was generated using the MagMax^{™} Sample Preparation System following the manufacturer's protocol (Thermo Fisher Scientific). Purified DNA was quantitated and normalized to 5 ng/µL.

TaqMan^{™} Copy Number Variation Assays were performed with crude lysates and purified DNA from 18 whole blood samples. The copy number variation assay Hs00010003_cn, 48DGV IDs was performed with the exemplary PCR composition of Example 1 in a 10 µL PCR with either 2 µL of crude lysate or 10 ng of purified DNA. Copy number was calculated by ΔΔCt method with RNase P gene as a normalizing gene and Applied Biosystems^{™} CopyCaller^{™} Software v2.1 was used for copy number calculation. FIG. 4 depicts the results with the bars on the left side indicating copy numbers from the crude lysate and the bars on the right side indicating copy numbers from corresponding purified DNA samples. The copy number variation result was comparative between crude lysate and purified DNA samples due to the high inhibitor tolerance of the exemplary PCR composition provided herein. Accordingly, the exemplary composition provided herein performs well in copy number variation assays using crude lysate samples. These results show that the exemplary composition provided herein can generate accurate copy number results using crude nucleic acid samples. PCR and copy number assay with the exemplary composition tolerates PCR inhibitors present in crude nucleic acid samples.

### Example 5: Comparison of varying concentrations of different antifoam agents

Fast SYBR^{®} Green Master Mix (Thermo Fisher Scientific) was purchased and used according to the manufacturer's instructions at a 1X concentration with additional components added as described below. 10pg/µL of template cDNA (made with Agilent's Universal Human Reference RNA and Applied Biosystem's High-Capacity cDNA Reverse Transcription Kit according to manufacturer's instructions) and 200nM of 18s gene target forward and reverse primers were added to the master mix. Varying concentrations (0%, 0.01%, 0.05%, and 0.1%) of either Antifoam 204 (Sigma) (FIG.5) or Antifoam SE-15 (Sigma) (FIG. 6) were added to the master mix prior to aliquotting 10-uL reactions/well in a 384-well microtiter plate (each of the four different antifoam concentrations tested in 96-well replicates). Prior to amplification, but after plating and centrifugation at 1000 rpm for 60 seconds, bubble formation within each reaction well was visually inspected. Amplification was then performed using the default fast cycling conditions for the ViiA 7 Real-Time PCR System (Applied Biosystems) according to the manufacturer's instructions. In FIG. 5 and FIG. 6, the horizontal and red curves in the graphs represent the fluorescence signal of the ROX passive reference dye while the green curves track SYBR^{®} Green dye fluorescence of the PCR target during thermal cycling.

As shown in FIG. 5, without the addition of Antifoam 204, the top-left plot shows that 87 bubbles were observed prior to cycling and that those bubbles vanished anywhere between cycle 0 - 36 (indicated by the increase in ROX fluorescence). With the addition of 0.01% of Antifoam 204, 60 bubbles were counted prior to PCR and appeared to have been eliminated prior to the first cycle of the reaction. With the addition of 0.05% and 0.1% of Antifoam 204, bubbles were not observed after plate centrifugation and did not affect the ROX passive signal.

As shown in FIG. 6, without the addition of Antifoam SE-15, the top-left plot shows that 92 bubbles were observed prior to cycling and that those bubbles vanished anywhere between cycle 0 - 40 (indicated by the increase ROX fluorescence). With the addition of 0.01% of Antifoam SE-15, 10 bubbles were counted prior to PCR and appeared to have been eliminated prior to the second cycle of the reaction. With the addition of 0.05% and 0.1% of Antifoam SE-15, bubbles were not observed after plate centrifugation and did not affect the ROX passive signal.

## Claims

1. A composition comprising:
(a) at least one DNA polymerase;
(b) at least one salt;
(c) at least one antifoam agent;
(d) at least one dNTP; and
(e) at least one dNTP derivative.

2. The composition of claim 1, wherein said DNA polymerase is a thermostable DNA polymerase, and optionally, wherein said thermostable DNA polymerase is selected from a Taq DNA polymerase; a Tne DNA polymerase; a Tma DNA polymerase; a Pfu DNA polymerase; a KOD DNA polymerase; a Tfl DNA polymerase; a Tth DNA polymerase; a Stoffel fragment DNA polymerase; a VENT^{™} DNA polymerase; a DEEPVENT^{™} DNA polymerase; and mutants, variants or derivatives thereof, preferably, wherein said thermostable DNA polymerase is a Taq-derived DNA polymerase.

3. The composition of claim 1, wherein said salt is selected from a potassium salt, a magnesium salt, a sodium salt, and any combination thereof.

4. The composition of claim 3, wherein said salt is at a total concentration of 5 mM to 200 mM.

5. The composition of claim 1, wherein said antifoam agent is a silicone-based antifoam agent, and optionally wherein said silicone-based antifoam agent is selected from XIAMETER^{®} AFE-1010, AFE-1430, AFE-1510, AFE-1520, AFE-2210, Antifoam A, Antifoam B, Antifoam C, Antifoam H-10, Antifoam SE-15, Antifoam SE-35, Antifoam SO-25, Antifoam Y-30, and Antifoam 289;
or wherein said antifoam agent is a non-silicone-based antifoam agent, and optionally wherein said non-silicone-based antifoam agent is selected from an organic sulfonate, a polyether, an organic phosphate, an acetylenic glycol, a fluorocarbon, a polyalkene polyamine, and a polyalkyleneimine compound.

6. The composition of claim 5, wherein said antifoam agent is at a concentration of 0.001% to 0.1%.

7. The composition of claim 1, wherein said dNTP is a dGTP, and wherein said dNTP derivative is a 7-deaza-dGTP (7-deaza-2-deoxy-dGTP).

8. The composition of claim 1, wherein said composition further comprises one or more PCR inhibitor blocking agent.

9. A method for performing a nucleic acid synthesis reaction or a PCR comprising:
(a) contacting the composition of any one of the previous claims with a biological sample or nucleic acid sample comprising a target polynucleotide with a target-specific primer to form a reaction mixture; and
(b) performing nucleic acid synthesis or PCR.

10. The method of claim 9, further comprising c) determining a genotype of the nucleic acid sample using the amplification product or c) determining copy number of the target polynucleotide using the amplification product.

11. The method of claim 10, wherein the run time of the PCR is decreased as compared to an equivalent PCR performed with a standard PCR reaction mix.

12. The method of claim 10, wherein the PCR is a simplex PCR or a multiplex PCR.

13. The method of claim 10, wherein the nucleic acid sample is a lysate, and optionally wherein the lysate is from blood or oral cells.

14. The method of claim 10, wherein the nucleic acid sample is from saliva, urine, or serum.

15. A kit comprising the composition of any one of claims 1 to 8, further comprising a primer pair and labeled probe all specific for PCR amplification and detection of a DNA target.

16. A kit comprising the composition of any one of claims 1 to 8, further comprising a control nucleic acid sample, and a primer pair specific for PCR amplification of a DNA target in the control nucleic acid sample.
